(19) Europäisches Patentamt
European Patent Office
Office européen des brevets

(11) EP 1 988 084 A1

(12) EUROPEAN PATENT APPLICATION
published in accordance with Art. 158(3) EPC

(43) Date of publication:
**05.11.2008 Bulletin 2008/45**

(21) Application number: **07713911.1**

(22) Date of filing: **01.02.2007**

(51) Int Cl.:
***C07D 257/10*** (2006.01) ***B01J 31/22*** (2006.01)
***B01J 37/08*** (2006.01) ***B01J 37/34*** (2006.01)
***C07F 1/08*** (2006.01) ***C07F 9/00*** (2006.01)
***C07F 13/00*** (2006.01) ***C07F 15/02*** (2006.01)
***C07F 15/04*** (2006.01) ***C07F 15/06*** (2006.01)

(86) International application number:
**PCT/JP2007/052157**

(87) International publication number:
**WO 2007/091616 (16.08.2007 Gazette 2007/33)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.02.2006 JP 2006030584**

(71) Applicant: **Sumitomo Chemical Company, Limited**
**Tokyo 104-8260 (JP)**

(72) Inventors:
- **KOSHINO, Nobuyoshi**
**Tsukuba-shi, Ibaraki 3050821 (JP)**
- **ISHIYAMA, Takeshi**
**Tsukuba-shi, Ibaraki 3050821 (JP)**
- **MATSUNAGA, Tadafumi**
**Tsukuba-shi, Ibaraki 3050821 (JP)**
- **HIGASHIMURA, Hideyuki**
**Tsukuba-shi, Ibaraki 3050045 (JP)**

(74) Representative: **Vossius & Partner**
**Siebertstrasse 4**
**81675 München (DE)**

(54) **METAL COMPLEX AND USE THEREOF**

(57) A polynuclear metal complex having in each molecule not only at least one large cyclic ligand having 5 to 15 coordinating atoms but also multiple metal atoms are subjected to any of heating treatment, radiation exposure treatment and electric discharge treatment, thereby providing a polynuclear metal complex exhibiting a ratio of weight loss by the treatment of 5 to 90 wt.% and a carbon content after the treatment of 5 wt.% or more.

EP 1 988 084 A1

## Description

TECHNICAL FIELD

The invention relates to a metal complex suitable as a catalyst. Further, the invention relates to a catalyst containing the metal complex.

BACKGROUND ART

Metal complexes act as catalysts (redox catalysts) in redox reactions involving electron transfer such as oxygen addition reactions, oxidative coupling reactions, dehydrogenation reactions, hydrogenation reactions and oxide decomposition reactions and are used for producing organic compounds or polymer compounds. Further, they are also used for various applications such as additives, reforming agents, batteries, and materials for sensors.

Particularly, among the metal complexes, as a redox reaction catalyst, if a multinuclear complex having two or more metal atoms which are assenbled one another to a certain extent is used, the redox reaction involving multiple electron transfer is made possibly and therefore, the reaction speed of the redox reaction can be increased or side reactions by radical species produced by one electron transfer can be suppressed (e.g., reference to Kenichi OYAIZU, Makoto YUASA, Surface 41(3), 22 (2003)). Further, it has been known that in an oxidation coupling reaction, radicals of two or more molecules can be produced simultaneously and thus not only the reaction speed is increased but also the reaction selectivity can be controlled.

Herein, the multinuclear complex is, as described in Michinori OKI, et al., ENCYCLOPAEDIA CHIMICA, section 1338 (Tokyo Kagaku Dojin, first edition (7th printing) issued on July 1, 2005), defined as a compound containing two or more metal atoms as central atoms in each complex molecule. As an example of the above-mentioned redox reaction catalyst, it is known to use a manganese-dinuclear complex as a catalyst (a hydrogen peroxide decomposition catalyst) for decomposing hydrogen peroxide into water and oxygen while suppressing generation of free radicals ( hydroxyl radical, hydroperoxyl radical, and the like).

DISCLOSURE OF THE INVENTION

However, in the case of using the manganese-dinuclear complex as disclosed in A. E. Boelrijk and G. C. Dismukes, Inorg. Chem. 2000, 39, 3020 as a hydrogen peroxide decomposition catalyst, the dinuclear complex is insufficient in the stability and particularly in the case of reaction performed in the presence of an acid or under heating, use of this catalyst causes a problem. In such a reaction using the multinuclear complex catalyst, it has been therefore desired to improve the stability to the presence of an acid or to heating.

An object of the invention is to provide a metal complex having high reaction activity as a redox catalyst, excellent in thermal stability, and suitable as a catalyst.

The inventors of the invention have made various investigations to find a catalyst having high reaction activity particularly as a redox catalyst and excellent in thermal stability and accordingly have completed this invention.

That is, the invention provides a metal complex suitable as a catalyst according to the following [1] to [12].

[1] A multinuclear metal complex having at least one macrocyclic ligand having 5 to 15 coordinating atoms and two or more metal atoms, wherein the metal complex is subjected to any of heating treatment, radiation exposure treatment, and electric discharge treatment, thereby the complex shows a ratio of weight loss by the treatment of 5 to 90 wt.% and a carbon content after the treatment of 5 wt.% or more.

[2] The metal complex according to [1], wherein the metal atoms are transition metal atoms belonging to Group 4 to Group 6 in the periodic stable.

[3] The metal complex according to [1] or [2], wherein the number of the metal atoms is 2 to 10.

[4] The metal complex according to one of [1] to [3], wherein the coordinating atoms are selected from a group consisting of a carbon atom, a nitrogen atom, an oxygen atom, a phosphorus atom, and a sulfur atom.

[5] The metal complex according to one of [1] to [4], wherein the macrocyclic ligand is a macrocyclic ligand defined by the following formula (I)

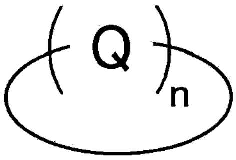 (I)

wherein Q denotes a divalent organic group containing one or more of coordinating atoms selected from a group consisting of a carbon atom, a nitrogen atom, an oxygen atom, a phosphorus atom, and a sulfur atom and n denotes an integer of 5 or more and 15 or less.

[6] The metal complex according to one of [1] to [5], wherein the treatment is a treatment at a temperature of 250°C or more and 1000°C or less.

[7] The metal complex according to one of [1] to [6], wherein the ratio of the weight loss by the treatment is 7 wt.% or more and 90 wt.% or less.

[8] The metal complex according to one of [1] to [7], wherein the carbon content of the multinuclear metal complex is 10 wt.% or more.

[9] The metal complex according to one of [1] to [8], wherein the metal complex has a peak in a range of 1550 to 1600 $cm^{-1}$ in a spectrum measured by laser Raman spectrometry at an excitation wavelength of 532 nm.

[10] The metal complex according to one of [1] to [9], wherein the half width of a signal of the metal complex having the maximum peak in a range of 396 eV to 404 eV in an N1s spectrum by X-ray photoelectric spectroscopy is 2 eV or more.

[11] The metal complex according to one of [1] to [10], wherein the half width of the signal of the metal complex having the maximum peak in a range of 396 eV to 404 eV in an N1s spectrum by X-ray photoelectric spectroscopy is 1.3 times as large as the half width of the signal of the multinuclear metal complex before the treatment.

[12] A metal complex showing a ratio of weight loss by the treatment of 5 to 90 wt. % and a carbon content after the treatment of 5 wt.% or more in the case of subjecting a mixture of (a) a multinuclear metal complex having at least one macrocyclic ligand containing 5 to 15 coordinating atoms and two or more metal atoms and (b) a carbon carrier or at least one organic compound selected from organic compounds having a boiling point or melting point of 250°C or more and more and organic compounds having a thermal polymerization starting temperature of 250°C or less to any of heating treatment, radiation exposure treatment, and electric discharge treatment.

[13] A composition containing the multinuclear metal complex according to one of [1] to [12] as well as a carbon carrier and/or a conductive polymer.

Further, the invention provides [14] a catalyst containing the multinuclear metal complex according to one of [1] to [12] and/or the composition according to [13].

BRIEF DESCRIPTION OF THE DRAWINGS

Fig. 1: A thermogravimetric analysis chart of a multinuclear metal complex (A)
Fig. 2: A thermogravimetric analysis chart of a multinuclear metal complex (B)
Fig. 3: A thermogravimetric analysis chart of a multinuclear metal complex (C)
Fig. 4: A thermogravimetric analysis chart of a multinuclear metal complex (D)
Fig. 5: A thermogravimetric analysis chart of a multinuclear metal complex (E)
Fig. 6: A thermogravimetric analysis chart of a multinuclear metal complex (F)
Fig. 7: A thermogravimetric analysis chart of a multinuclear metal complex (G)
Fig. 8: A thermogravimetric analysis chart of a multinuclear metal complex (H)
Fig. 9: A laser Raman spectrum of a multinuclear metal complex (A) and a modified complex (A) of Example 10
Fig. 10: An N1s spectrum of a multinuclear metal complex (E) and a modified complex (E) of Example 11 by X-ray photoelectric analysis
Fig. 11: A C1s spectrum of a multinuclear metal complex (E) and a modified complex (E) of Example 10 by X-ray photoelectric analysis
Fig. 12: Results of a hydrogen peroxide decomposition test of Example 19 and Comparative Example 9
Fig. 13: A GPC analysis chart of Example 20

BEST MODE FOR CARRYING OUT THE INVENTION

A metal complex of the invention is a metal complex showing a ratio of weight loss by the treatment of 5 to 90 wt.% and a carbon content after the treatment of 5 wt.% or more in the case of subjecting a multinuclear metal complex having at least one macrocyclic ligand having 5 to 15 coordinating atoms and two or more metal atoms, or a mixture of the multinuclear metal complex and an organic compound to a specific treatment.

The inventors of the invention have found that in the case of metal coordinated polymers disclosed so far, no good reaction activity can be obtained since they are in a mononuclear state that only one mental ion is coordinated at a coordination site existing in polymer side chains and therefore, assembly of the metal atoms are insufficient and also have obtained an innovative finding that in order to improve the catalytic activity, the coordination structure of assembled metal atoms should be stabilized by modifying the multinuclear metal complex by heat treatment or the like.

On the other hand, the inventors of the invention have found that the above-mentioned metal complex is useful as an innovative catalyst for solving the problems described above, by combining the findings that with respect to catalysts obtained by heat treatment of multinuclear metal complexes disclosed so far, since the ligands themselves are easy to be evaporated, the coordination structures of metal atoms in the metal complexes are broken by heat treatment process, and the above-mentioned finding.

Herein, the metal atoms may be those having no electric charge or ions bearing electric charge. The coordinating atoms are, as described in page 966, Ryogo KUBO, et al., Encyclopedia of Physics and Chemistry 4th ed. (issued on Jan., 10, 1991 Iwanami Shoten), atoms having unshared electron pairs to donate electrons to an unoccupied orbital of the metal atoms and bonded with the metal atoms by coordination bonds. Further, a macrocyclic ligand having the coordinating atoms is a cyclic organic compound capable of forming a stable coordinating structure by showing macrocyclic effect described in page 1323 to 1324 of Michinori OKI, et al., ENCYCLOPAEDIA CHIMICA (issued on July 1, 2005, Tokyo Kagaku Dojin). The macrocyclic ligand of the invention may include those having a structure formed by joining macrocyclic organic compounds as described above and having 5 to 15 coordinating atoms in total.

Herein, the above-mentioned metal atoms are preferably transition metals belonging to Group 4 to Group 6 of the periodic table (long form ).

Examples are metal atoms selected from a group consisting of scandium, titanium, vanadium, chromium, manganese, iron, cobalt, nickel, copper, yttrium, zirconium, niobium, molybdenum, technetium, ruthenium, rhodium, palladium, silver, lanthanum, cerium, praseodymium, neodymium, promethium, samarium, europium, gadolinium, terbium, dysprosium, holmium, erbium, thulium, ytterbium, lutetium, hafnium, tantalum, tungsten, rhenium, osmium, iridium, platinum and gold.

More preferable examples are metal atoms selected from a group consisting of scandium, titanium, vanadium, chromium, manganese, iron, cobalt, nickel, copper, yttrium, zirconium, niobium, molybdenum, silver, lanthanum, cerium, praseodymium, neodymium, promethium, samarium, europium, gadolinium, terbium, dysprosium, holmium, erbium, thulium, ytterbium, lutetium, hafnium, tantalum, and tungsten and even more preferable examples are metal atoms selected from a group consisting of scandium, titanium, vanadium, chromium, manganese, iron, cobalt, nickel, copper, zirconium, niobium, molybdenum, tantalum, and tungsten.

Among these atoms, more particularly preferable examples are metal atoms selected from a group consisting of vanadium, chromium, manganese, iron, cobalt, nickel, and copper and even more particularly preferable examples are metal atoms selected from a group consisting of manganese, iron, cobalt, nickel, and copper.

The multinuclear metal complex to be used in the invention has two or more metal atoms selected from the above examples and the number of the metal atoms is preferably 30 or less, more preferably 2 to 5, furthermore preferably 2 to 3, and even more preferably 2.

The macrocyclic ligand in the multinuclear metal complex may be those which have 5 to 15 coordinating atoms capable of forming coordination bonds with metal atoms or more than one macrocyclic ligands having 5 to 15 coordinating atoms in total. The total of the coordinating atoms of the macrocyclic ligand is preferably 5 to 12, more preferably 6 to 10, and even more preferably 6 to 8.

Further, the coordinating atoms may be electrically neutral or charged ions.

The coordinating atoms may be selected from a carbon atom, a nitrogen atom, an oxygen atom, a phosphorus atom and a sulfur atom and those selected coordinating atoms may be same or different one another. They are more preferably a nitrogen atom, an oxygen atom, and a sulfur atom and even more preferably a nitrogen atom and an oxygen atom. Particularly, if the macrocyclic ligand has a nitrogen atom and/or an oxygen atom as coordinating atoms, in the treatment described later, the spatial arrangement of metal atoms of the initial multinuclear metal complex is easy to be maintained and a metal atom accumulation state preferable as a catalyst can be retained and therefore, it is preferable.

Further, the molecular weight of the above-mentioned macrocyclic ligand is preferably 160 or more. It is more preferably not less than 250, furthermore preferably not less than 300, and even more preferably not less than 400. In the case the molecular weight is less than 160 or the coordinating atoms are 4 or less, the boiling point is low or the complex structure itself tends to be unstable when the ligand is coordinated with metal atoms. In this case, when the

above-mentioned treatments is carried out, since the organic components are easy to be evaporated, the weight decrease ratio sometimes becomes significant or the multinuclear complex structure may sometimes be broken and in both cases, the catalytic activity of the invention is deteriorated. The upper limit of the molecular weight is preferably not higher than 3000. It is more preferably not higher than 2000 and even more preferably not higher than 1000. In the case the molecular weight of the macrocyclic ligand is high, the multinuclear complex becomes inhomogeneous.

The macrocyclic ligand is, as described above, either a cyclic organic compound or a compound containing multiple cyclic organic compounds are connected to one another and a preferable example is a cyclic organic compound defined by the following formula (I) or a compound containing two bonded cyclic organic compounds as defined by the following formula (II):

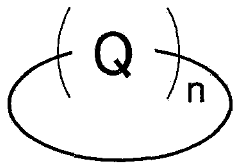

(I)

wherein Q denotes a divalent group containing one or more coordinating atoms selected from a carbon atom, a nitrogen atom, an oxygen atom, a phosphorus atom, and a sulfur atom; n denotes an integer of 5 to 15; multiple atoms for Q may be same or different one another: and

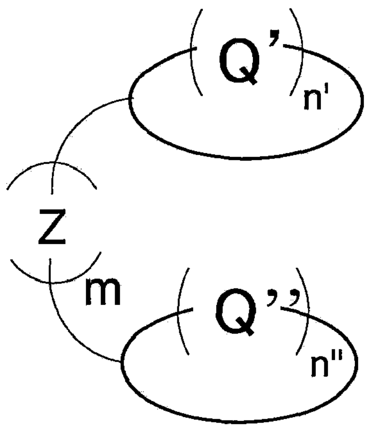

(II)

wherein Q' and Q" independently denote same as Q in the formula (I); n' and n" independently denote an integer equal to 1 or higher and the total number of n' and n" is 5 to 15; Z denotes a divalent group optionally containing coordinating atoms and a group for bonding one atom among multiple atoms for Q' and one atom among multiple atoms for Q"; m denotes an integer of 0 to 7; and in the case there are multiple groups for Z, the groups may be same or different one another.

Examples of Q, Q', and Q" in the general formulas (I) and (II) may be an oxy group, a thioxy group, an amino group, and an imino group (the imino group include a tertiary imino group having an alkyl group having 1 to 50 carbon atoms or an aromatic group having 2 to 60 carbon atoms); an alkylene group, an alkenylene group, and a divalent aromatic group (including an aromatic heterocyclic group) containing these groups; and an alkylene group, an alkenylene group, and a divalent aromatic group (including an aromatic heterocyclic group) containing these groups having a monovalent group having coordinating atoms such as an amino group, a hydroxyl group, and a thiol group.

Further, the above-mentioned alkylene group and divalent aromatic group (including a divalent aromatic heterocyclic group) may have a substituent such as an alkyl group, an aryl group, an aralkyl group, a nitro group, a cyano group, or a halogen atom.

Examples of the above-mentioned alkyl group may be linear, branched, and cyclic alkyl groups having about 1 to 50 carbon atoms such as a methyl group, an ethyl group, a propyl group, an isopropyl group, a butyl group, an isobutyl group, a tert-butyl group, a pentyl group, a cyclopentyl group, a hexyl group, a cyclohexyl group, a norbonyl group, a nonyl group, a decyl group, and a 3,7-dimethyloctyl group.

Examples of the above-mentioned aryl group may be aryl groups having about 6 to 50 carbon atoms such as a phenyl group, a tolyl group, a biphenyl group, a naphthyl group, am anthralyl group, a phenanthryl group, and a

terphenyl group.

Examples of the above-mentioned aralkyl group may be aralkyl groups having about 7 to 50 carbon atoms such as a phenylmethyl group, a 1-phenylethyl group, a 2-phenylethyl group, a 1-phenyl-1-propyl group, a 1-phenyl-2-propyl group, a 2-phenyl-2-propyl group, a 1-phenyl-3-propyl group, a 1-phenyl-4-butyl group, a 1-phenyl-5-pentyl group, and a 1-phenyl-6-hexyl group.

Further, the above-mentioned halogen atom is selected from a fluorine atom, a chlorine atom, a bromine atom, and an iodine atom.

Preferable among the above-mentioned examples of Q, Q' , and Q" are divalent organic groups containing a nitrogen atom or an oxygen atom as the coordinating atom as defined by the following (Q-1) to (Q-14).

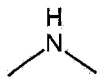
(Q-1)

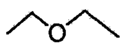
(Q-2)

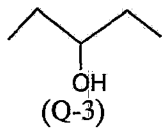
(Q-3)

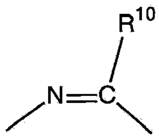
(Q-4)

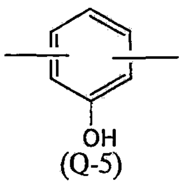
(Q-5)

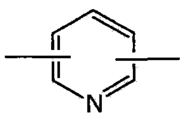
(Q-6)

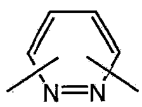
(Q-7)

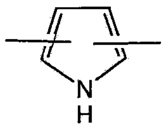
(Q-8)

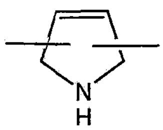
(Q-9)

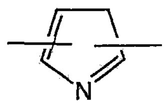
(Q-10)

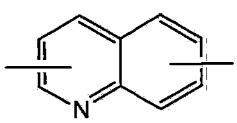
(Q-11)

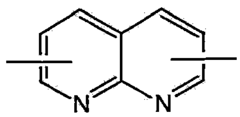
(Q-12)

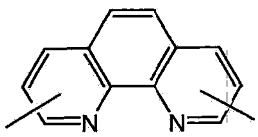
(Q-13)

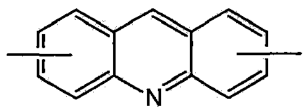
(Q-14)

In the above-mentioned formulas, $R^{10}$ denotes a hydrogen atom or a monovalent organic group and typical examples are, as described above, an alkyl group having 1 to 50 carbon atoms or an aromatic group having 2 to 60 carbon atoms.

The hydroxyl group in the above-mentioned (Q-3) and (Q-5) may become a phenolato group by releasing proton and may be coordinated with metal atoms.

Examples of Z in the formula (II) may be a divalent group such as an (un) substituted alkylene group, an (un) substituted alkenylene group, an (un)substituted aromatic group, an oxy group, a thioxy group, an imino group (including a tertiary imino group having an alkyl group having 1 to 50 carbon atoms or an aromatic group having 2 to 60 carbon atoms), and may be a divalent group formed by bonding divalent groups selected from these groups.

Examples of the alkylene group may be linear, branched, and cyclic alkylene groups having about 1 to 20 carbon atoms in total such as a methylene group, an ethylene group, a 1,1-propylene group, a 1,2-propylene group, a 1,3-propylene group,a 2,4-butylene group, a 2,4-dimethyl-2,4-butylene group, a 1,2-cyclopentylene group, and a 1,2-cyclohexylene group.

Further, the alkylene group may have a substituent such as an alkoxy group having 1 to 50 carbon atoms, a nitro group, a cyano group, or a halogen atom.

Examples of the alkoxy group may be alkoxy groups having about 1 to 50 carbon atoms such as a methoxy group, an ethoxy group, a propoxy group, a hexyloxy group, and a decyloxy group.

The halogen atom may be selected from a fluorine atom, a chlorine atom, a bromine atom, and an iodine atom.

The (un)substituted aromatic group in Z of the general formula (II) is a divalent group derived from an aromatic compound by removing two hydrogen atoms.

Examples of the aromatic compound may be aromatic compounds having about 2 to 60 carbon atoms in total such as benzene, naphthalene, anthracene, phenanthrene, tetracene, biphenyl, biphenylene, furan, dibenzofuran, thiophene, dibenzothiophene, pyridine, bipyridine, phenanthroline, and xanthene.

The aromatic group may have a substituent such as an alkyl group, an aralkyl group, an alkoxy group, a nitro group, a cyano group, or a halogen atom.

Herein, examples of the alkyl group, alkoky group, aralkyl group, and a halogen atom may be same as those exemplified as substituent for the above-mentioned alkylene group.

The reference character m in the general formula (II) denotes an integer of 0 to 7. It is preferably 1 to 5 and more preferably 1 to 3. The expression that m is 0 means that a cyclic compound consisting of multiple groups for Q' and a cyclic compound consisting of multiple groups for Q" are directly bonded.

Examples of the macrocyclic ligand defined by the above-mentioned formula (I) or (II) may be compounds defined by the following formulas (III-a) to (III-h). Preferable examples are compounds defined by the following formulas (III-a) to (III-e), more preferable examples are compounds defined by the following formulas (III-a) to (III-d), and even more preferable examples are compounds defined by the following formula (III-a).

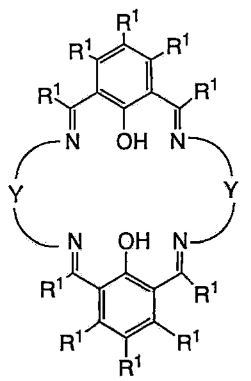
(III-a)

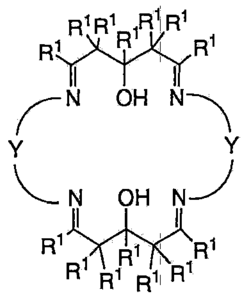
(III-b)

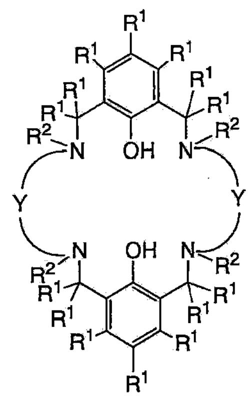
(III-c)

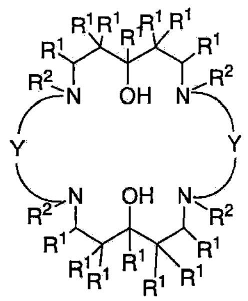
(III-d)

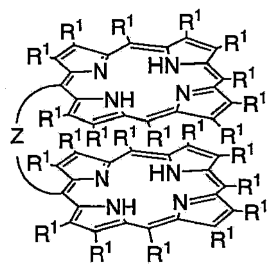
(III-e)

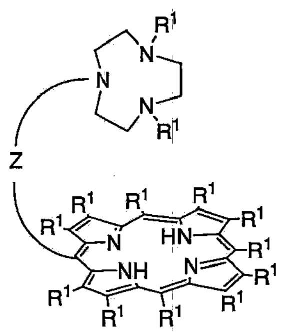
(III-f)

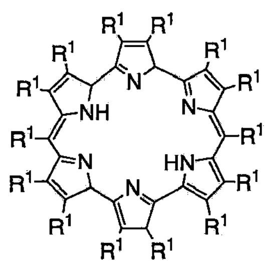
(III-g)

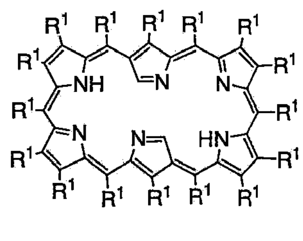
(III-h)

In the formulas (III-a) to (III-h), $R^1$ and $R^2$ denote a hydrogen atom or a substituent and two groups for $R^1$ bonded to neighboring two atoms or $R^1$ and $R^2$ may be bonded each other and multiple groups for $R^1$ and $R^2$ may be same or different. In the formula (III-a) to (III-d), Y denotes a divalent group forming a macrocyclic ligand and specific

examples are same as those for Z in the above-mentioned formula (II). Further, in the formulas (III-e) to (III-f), Z denotes the same in formula (II). In the formulas (III-a) to (III-h), $R^1$ and $R^2$ denote a hydrogen atom or a substituent and examples of the substituent are a functional group such as a halogen atom, a hydroxyl group, a carboxyl group, a mercapto group, a sulfonic acid group, a nitro group, a phosphonic acid group, or a silyl group having an alkyl group having 1 to 3 carbon atoms; an alkyl group having about 1 to 50 carbon atoms in total; an alkoxy group having about 1 to 50 carbon atoms in total; an aromatic group having about 2 to 60 carbon atoms in total; and the like.

Herein, examples of the alkyl group and alkoxy group are same as those groups exemplified as the substituents for Q, Q', Q", and Z in the above-mentioned formulas (I) and (II).

Further, examples of the aromatic group may be aromatic groups having about 2 to 60 carbon atoms in total such as a phenyl group, a methylphenyl group, a naphthyl group, a pyridyl group, a furazyl group, an oxazolyl group, an imidazolyl group, a pyrazolyl group, a pyrazyl group, a pyrimidyl group , a pyridazyl group, and a benzoimidazolyl group.

These alkyl group, alkoxy group, and aromatic group may have the above-mentioned functional groups as a substituent.

Particularly preferable examples for $R^1$ and $R^2$ of the formulas (III-a) to (III-h) are a hydrogen atom, a methyl group, a phenyl group, a methylphenyl group, a naphthyl group, and a pyridyl group and if such groups exist as $R^1$ and $R^2$, the catalytic activity of the metal complex to be obtained by treatment described later can be improved and therefore, it is preferable.

Y in the above-mentioned formulas (III-a) to (III-d) is defined same as Z in the above-mentioned formula (II) and particularly, an (un)substituted alkylene group, an (un)substituted cycloalkylene group, and a divalent aromatic group are preferable.

The alkylene group is a residual group formed by removing two hydrogen atoms from a single or different carbon atoms from an aliphatic hydrocarbon and examples may be a methylene group, an ethylene group, a propylene group, a butylene group, a pentylene group, a hexylene group, an isopropylene group, an isobutylene group, and the like.

The cycloalkylene group is a residual group formed by removing two hydrogen atoms from a single or different carbon atoms from an alicyclic hydrocarbon and examples may be a cyclopropylene group, a cyclobutylene group, a cyclopentylene group, a cyclohexylene group, a cyclopentylidene group, a cyclohexylidene group, and the like.

The divalent aromatic group is a residual group formed by removing two hydrogen atoms from a single or different carbon atoms from an aromatic compound. Examples of the aromatic compound may be benzene, naphthalene, anthracene, tetracene, biphenyl and the like.

Preferable examples for Y are an ethylene group, a propylene group, an isopropylene group, a butylene group, a phenylene group, a naphthylene group, an anthracenylene group, a cyclobutylene group, a cyclopentylene group, and a cyclohexylene group; more preferable examples are an ethylene group, a propylene group, a butylene group, a phenylene group, a cyclohexylene group, a naphthylene group, an anthracenylene group, and a cyclohexylene group; furthermore preferable examples are an ethylene group, a propylene group, a phenylene group, a naphthylene group, and a cyclohexylene group; and even more preferably a propylene group and a phenylene group.

Herein, examples of the macrocyclic ligand having the structure defined by the above-mentioned formula (III-a) are defined by the following formulas (III-a-1) to (III-a-6). Among these, those defined by the following formulas (III-a-1) to (III-a-4) are especially preferable.

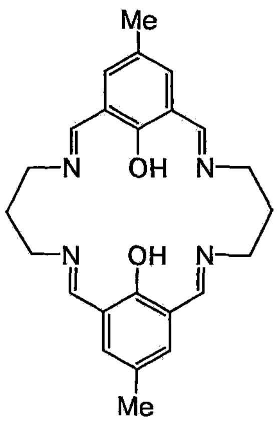

(III-a-1)

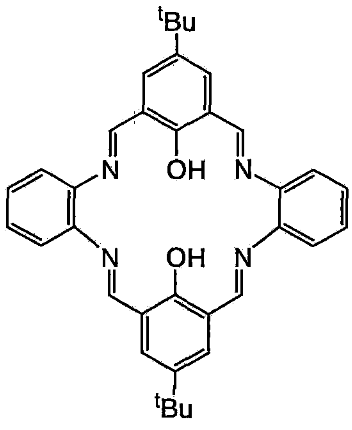

(III-a-2)

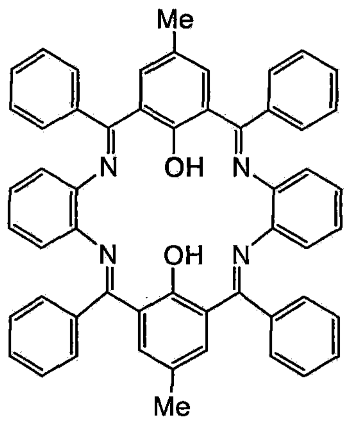

(III-a-3)

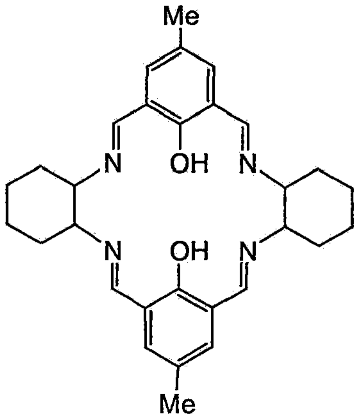

(III-a-4)

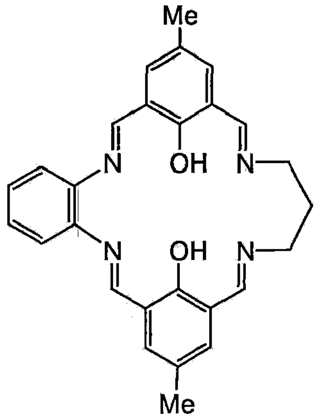

(III-a-5)

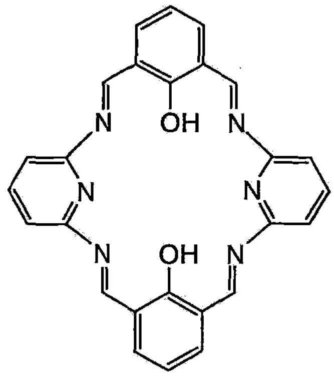

(III-a-6)

Examples of the macrocyclic ligand having the structure defined by the above-mentioned formula (III-b) are defined by the following formulas (III-b-1) to (III-b-2).

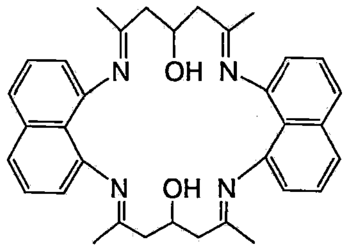

(III-b-1)

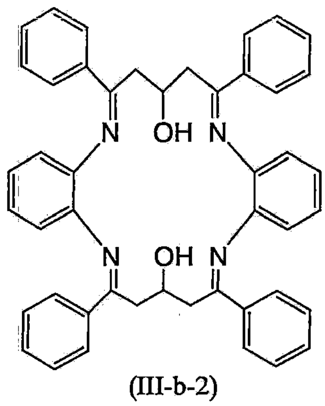

(III-b-2)

Examples of the macrocyclic ligand having the structure defined by the above-mentioned formula (III-c) are defined by the following formulas (III-c-1) to (III-c-3).

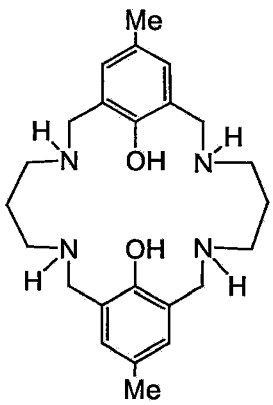

(III-c-1)

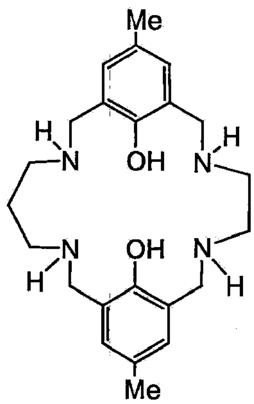

(III-c-2)

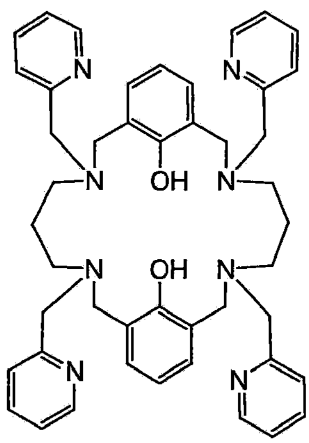

(III-c-3)

Examples of the macrocyclic ligand having the structure defined by the above-mentioned formula (III-d) are defined by the following formulas (III-d-1) to (III-d-2).

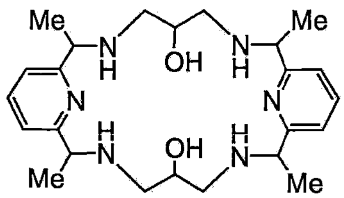

(III-d-1)

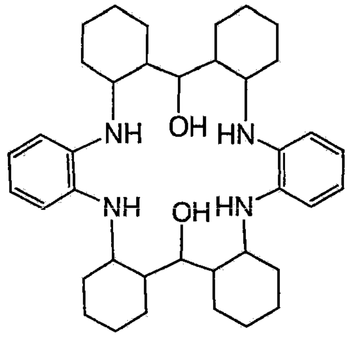

(III-d-2)

Examples of the macrocyclic ligand having the structure defined by the above-mentioned formula (III-e) are defined by the following formulas (III-e-1) to (III-e-3).

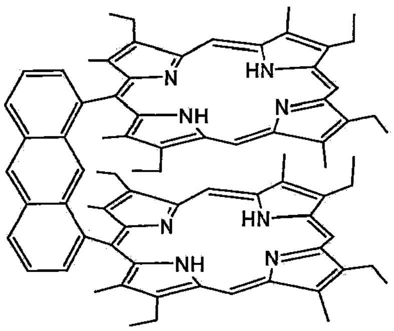

(III-e-1)

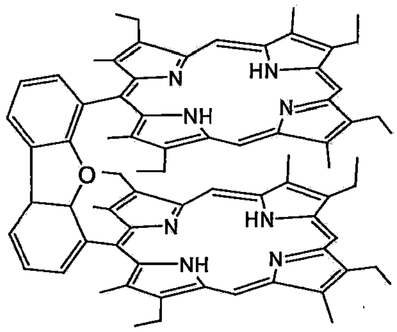

(III-e-2)

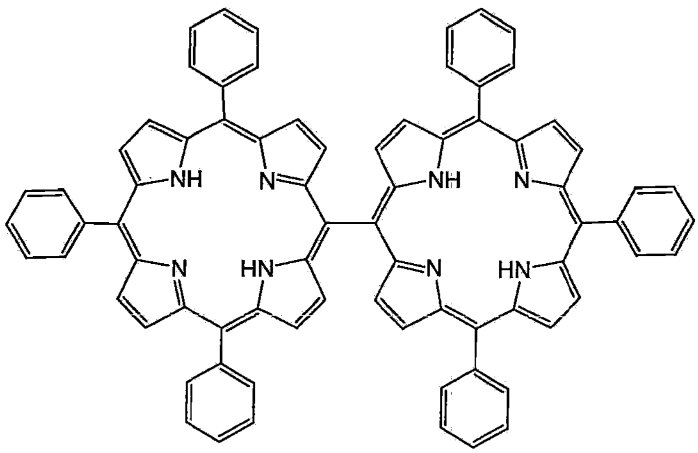

(III-e-3)

Examples of the macrocyclic ligand having the structure defined by the above-mentioned formula (III-f) are defined by the following formulas (III-f-1) to (III-f-2).

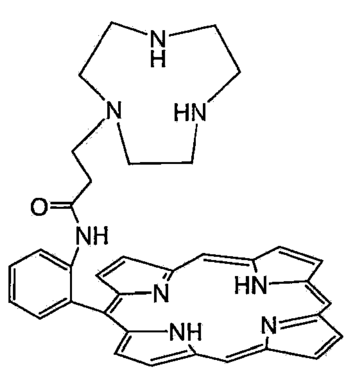

(III-f-1)

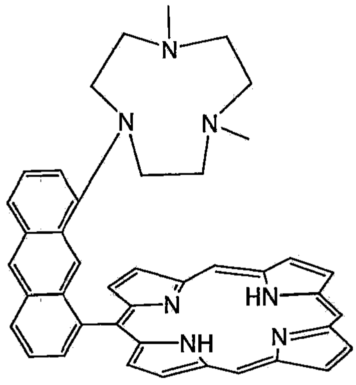

(III-f-2)

Examples of the macrocyclic ligand having the structure defined by the above-mentioned formula (III-g) are defined by the following formulas (III-g-1) to (III-g-2).

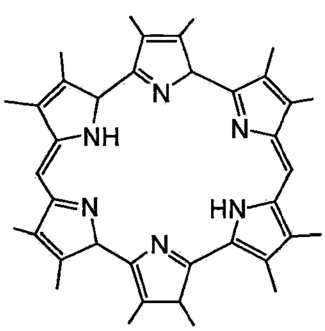

(III-g-1)

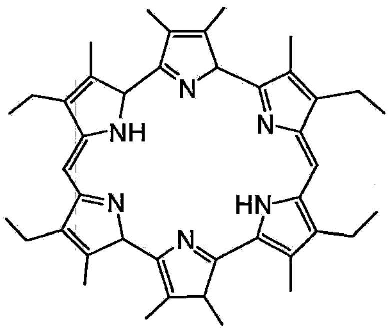

(III-g-2)

Examples of the macrocyclic ligand having the structure defined by the above-mentioned formula (III-h) are defined by the following formulas (III-h-1) to (III-h-2).

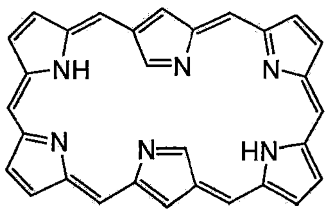

(III-h-1)

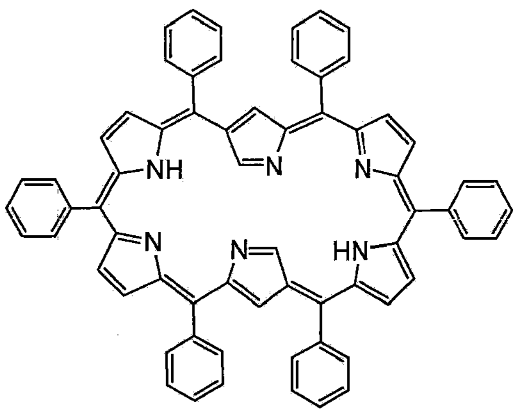

(III-h-2)

The macrocyclic ligand in the multinuclear metal complex of the invention is preferably compounds defined by the above exemplified formulas (I) and (II) and particularly preferably compounds defined by the formula (I). The multinuclear metal complex of the invention indispensably include two or more metal atoms and at least one macrocyclic ligand and may have other ligands in addition to the macrocyclic ligand. Other ligands may be ionic or electrically neutral compounds and in the case the multinuclear metal complex includes more than one other ligands, these ligands may be same or different.

Examples of the electrically neutral compound for the above-described another ligand may include nitrogen atom-containing compounds such as ammonia, pyridine, pyrrole, pyridazine, pyrimidine, pyrazine, 1,2,4-triazine, pyrazole, imidazole, 1,2,3-triazole, oxazole, isoxazole, 1,3,4-oxadiazole, thiazole, isothiazole, indole, indazole, quinoline, isoquinoline, phenantrizine, cinnoline, phthalazine, quinazoline, quinoxaline, 1,8-naphthylidine, acridine, 2,2'-bipyridine, 4,4'-bipyridine, 1,10-phenanthroline, ethylenediamine, propylenediamine, phenylenediamine, cyclohexanediamine, pyridine-N-oxide, 2,2'-bipyridine-N,N'-dioxide, oxamide, dimethyl glyoxime, and o-aminophenol; oxygen-containing compounds such as water, phenol, oxalic acid, catechol, salicylic acid, phthalic acid, 2,4-pentanedione, 1,1,1-trifluoro-2,4-pentanedione, hexafluoropentanedione, 1,3-diphenyl-1,3-propanedione, and 2,2'-binaphthol; sulfur-containing compounds such as dimethyl sulfoxide and urea; and phosphorus-containing compounds such as 1,2-bis(dimethylphosphino) ethane and 1,2-phenylenebis(dimethylphosphine).

Preferable examples are ammonia, pyridine, pyrrole, pyridazine, pyrimidine, pyrazine, 1,2,4-triazine, pyrazole, imidazole, 1,2,3-triazole, oxazole, isoxazole, 1,3,4-oxadiazole, indole, indazole, quinoline, isoquinoline, phenantrizine, cinnoline, phthalazine, quinazoline, quinoxaline, 1,8-naphthylidine, acridine, 2,2'-bipyridine, 4,4'-bipyridine, 1,10-phenanthroline, ethylenediamine, propylenediamine, phenylenediamine, cyclohexanediamine, pyridine-N-oxide, 2,2'-bipyridine-N,N'-dioxide, oxamide, dimethyl glyoxime, o-aminophenol, water, phenol, oxalic acid, catechol, salicylic acid, phthalic acid, 2,4-pentanedione, 1,1,1-trifluoro-2,4-pentanedione, hexafluoropentanedione, 1,3-diphenyl-1,3-propanedione, and 2,2'-binaphthol; and more preferable examples are ammonia, pyridine, pyrrole, pyridazine, pyrimidine, pyrazine, 1,2,4-triazine, pyrazole, imidazole, 1,2,3-triazole, oxazole, isoxazole, 1,3,4-oxadiazole, indole, indazole, quinoline, isoquinoline, phenantrizine, cinnoline, phthalazine, quinazoline, quinoxaline, 1,8-naphthylidine, acridine, 2,2'-bipyridine, 4,4'-bipyridine, 1,10-phenanthroline, ethylenediamine, propylenediamine, phenylenediamine, cyclohexanediamine, pyridine-N-oxide, 2,2'-bipyridine-N,N'-dioxide, o-aminophenol, phenol, catechol, salicylic acid, phthalic acid, 1,3-diphenyl-1,3-propanedione, and 2,2'-binaphthol.

Particularly more preferable examples among them are pyridine, pyrrole, pyridazine, pyrimidine, pyrazine, pyrazole, imidazole, oxazole, indole, quinoline, isoquinoline, acridine, 2,2'-bipyridine, 4,4'-bipyridine, 1,10-phenanthroline, phenylenediamine, pyridine-N-oxide, 2,2'-bipyridine-N,N'-dioxide, o-aminophenol, and phenol.

Further, examples of a ligand having anionic property are a hydroxyl ion, a peroxide, a superoxide, a cyanide ion, a thiocyanate ion; halide ions such as a fluoride ion, a chloride ion, a bromide ion, and an iodide ion; a sulfate ion, a nitrate ion, a carbonate ion, a perchlorate ion, a tetrafluoroborate ion; tetraaryl borate ions such as a tetraphenyl borate ion; a hexafluorophosphate ion, a methanesulfonate ion, a trifluoromethanesulfonate ion, a p-toluenesulfonate ion, a benzenesulfonate ion, a phosphate ion, a phosphite ion, an acetate ion, a trifluoroacetate ion, apropionate ion, abenzoate ion, a hydroxyl ion, metal oxide ions, a methoxide ion, and ethoxide ion and the like.

Preferable examples are a hydroxyl ion, a sulfate ion, a nitrate ion, a carbonate ion, a perchlorate ion, a tetrafluoroborate ion, a tetraphenyl borate ion, a hexafluorophosphate ion, a methanesulfonate ion, a trifluoromethanesulfonate ion, a p-toluenesulfonate ion, a benzenesulfonate ion, a phosphate ion, an acetate ion, a trifluoroacetate ion, and a hydroxyl ion and particularly preferable examples among them are a hydroxyl ion, a sulfate ion, a nitrate ion, a carbonate ion, a tetraphenyl borate ion, a trifluoromethanesulfonate ion, a p-toluenesulfonate ion, an acetate ion, and a trifluoroacetate ion.

Further, ions exemplified above as a ligand having an anionic property may be a counter ion electrically neutralizing the multinuclear metal complex of the invention.

Further, the multinuclear complex of the invention may sometimes include a counter ion having a cationic property to keep the electric neutrality. Examples of the counter ion having the cationic property may be alkali metal ions, alkaline earth metal ions; tetraalkylammonium ions such as a tetra(n-butyl)ammonium ion and a tetraethylammonium ion; and tetraarylphosphonium ions such as a tetraphenylphosphonium ion and specific examples are a lithium ion, a sodium ion, a potassium ion, a rubidium ion, a cesium ion, a magnesium ion, a calcium ion, a strontium ion, a barium ion, a tetra(n-butyl)ammonium ion, a tetraethylammonium ion, and a tetraphenylphosphonium ion and more preferable examples are a tetra(n-butyl)ammonium ion, a tetraethylammonium ion, and a tetraphenylphosphonium ion.

Particularly preferable among them are, as a counter ion having a cationic property, a tetra(n-butyl)ammonium ion and a tetraethylammonium ion.

Next, the treatment condition of the multinuclear metal complex of the invention will be described in detail.

The multinuclear metal complex to be used for the treatment may be one kind of multinuclear metal complex alone or two or more kinds of multinuclear metal complexes.

As pretreatment for the treatment, the multinuclear metal complex is particularly preferable to be dried at a

temperature of 15°C or higher and 200°C or lower under reduced pressure of 10 Torr or lower for 6 hours or longer. The pretreatment may be carried out using a vacuum drier or the like.

The atmosphere for carrying out the treatment of the multinuclear metal complex is preferably in the presence of hydrogen, helium, nitrogen, ammonia, oxygen, neon, argon, krypton, xenon, acetonitrile, or a gas mixture of these gases.

It is preferably in the presence of hydrogen, helium, nitrogen, ammonia, oxygen, neon, argon, or a gas mixture of these gases.

The pressure for the treatment may be properly changed in the treatment to be selected.

First, the heating treatment will be described specifically.

The temperature at the time of heating treatment for the multinuclear metal complex is not particularly limited if it is proper for adjusting a ratio of weight loss to be 5 wt.% or more.

The treatment temperature for the heating treatment is preferably not lower than 250°C, more preferably not lower than 300°C, furthermore preferably not lower than 400°C, and even more preferably not lower than 500°C. Further, the upper limit of the temperature for the heat treatment is also not particularly limited if it is proper for adjusting a ratio of weight loss to be 5 wt.% or more and it is preferably not higher than 1200°C, more preferably not higher than 1000°C, and even more preferably not higher than 800°C.

The treatment time for the heating treatment may be set properly depending on the above-mentioned gas to be used, temperature, and the like and in the state that the above-mentioned gas is tightly closed or ventilated, the temperature is gradually increased from room temperature to an aimed temperature and thereafter, it may be decreased immediately. Particularly, it is preferable to keep the temperature after the temperature reaches the aimed temperature since the multinuclear complex can be gradually modified and the durability can be improved more. The retention time after the temperature reaches the aimed temperature is preferably 1 to 100 hours, more preferably 1 to 40 hours, furthermore preferably 2 to 10 hours, and even more preferably 2 to 3 hours.

An apparatus for the heating treatment is not either particularly limited and an oven, a furnace, an IH hot plate, and the like can be exemplified. Further, if the amount of the multinuclear complex to be subjected to the heating treatment is about several tens of milligrams, in general, a furnace of a thermal analyzer to be used for thermal analysis can be employed. If a thermogravitational analyzer is used among thermal analyzers, the heating treatment can be stopped while the ratio of the weight loss is monitored and when a desired ratio of the weight loss is achieved and thus the heating treatment of the invention can easily be preformed.

With respect to the metal complex of the invention, it is supposed that the multinuclear metal complex forms a condensate by reaction of the macrocyclic ligands one another along with low weight molecule isolation and according weight loss by the above-mentioned heating treatment and the coordination structure is stabilized in the condensate. With respect to treatments for substituting the heating treatment, the same effect can be caused if the treatment can cause the ratio of the weight loss in the above-mentioned range.

Examples of the treatments for substituting the heating treatment may be selected from methods of radiating electromagnetic waves or particle beams such as a-ray, β-ray, neutron beam, electron beam, γ-ray, X-ray, vacuum ultraviolet ray, ultraviolet ray, visible ray, infrared ray, microwave, electric wave, laser and the like; and electric discharge treatment such as corona discharge treatment, glow discharge treatment, plasma treatment (including low temperature plasma treatment).

Preferable treatment among them may be treatment of radiating radiation beam selected from X-ray, electron beam, ultraviolet ray, visible ray, infrared ray, microwave, and laser and low temperature plasma treatment. More preferable treatment may be a method of radiating radiation beam selected from ultraviolet ray, visible ray, infrared ray, microwave, and laser.

These methods may be carried out according to instruments and treatment methods to be used generally for surface reforming treatment of polymer films and for examples, methods disclosed in a literature (Adhesion Society of Japan, "Chemistry of Surface Analysis, Reformation", issued by Nikkan Kogyo Shimbun on Dec. 19 2003), etc. can be employed.

Herein, at the time of carrying out the above-mentioned beam radiation treatment or discharge treatment, the condition may be arbitrarily set to adjust the ratio of weight loss by the treatment in a range of 5 wt.% to 90 wt.% and the carbon content after the treatment of 5 wt.% or more and preferable treatment time is within 10 hours, more preferably within 3 hours, furthermore preferably within 1 hour, and even more preferably within 30 minutes.

Any treatment of the heating treatment, beam radiation treatment, and discharge treatment is carried out as described above to an extent that the ratio of weight loss becomes not lower than 5 wt.%, preferably not lower than 10 wt.%, more preferably not lower than 15 wt.%, furthermore preferably not lower than 20 wt.%, and even more preferably not lower than 25 wt.% to obtain the metal complex of the invention.

On the other hand, in the case the weight is greatly decreased at the time of heating treatment, beam radiation treatment, electromagnetic wave radiation treatment, or discharge treatment, the decomposition of the multinuclear structure of the complex becomes too significant and therefore, it is not preferable. The upper limit of the ratio of weight

loss is preferably not higher than 80 wt.%, more preferably not higher than 70 wt.%, and even more preferably not higher than 60 wt.%.

Further, the metal complex of the invention has a carbon content of 5 wt.% or higher by elemental analysis. The carbon content is preferably not less than 10 wt.%, more preferably not less than 20 wt.%, furthermore preferably not less than 30 wt.%, and even more preferably not less than 40 wt.%. As the carbon content of the treated product is higher, the multinuclear structure is more stabilized and as the carbon content is higher, the stability is more improved and therefore, a high content is preferable.

As described above, with respect to the metal complex of the invention, it is supposed that macrocyclic ligands are reacted with one another by the treatment, that is, macrocyclic ligands are condensed involving low molecule isolation of the macrocyclic ligands and the metal atoms maintain the spatial arrangement in the produced modified ligand approximately same as that of the multinuclear metal complex before the treatment. Herein, if the macrocyclic ligand after the treatment is in a state of condensed and bonded in graphene-like strucurre , the thermal stability is improved and therefore, it is preferable. Further, in the case the modified multinuclear metal complex of the invention is used for a catalyst layer of a fuel cell, another effect of improving the conductivity is also caused. Existence of such graphene-like structure can be confirmed based on the existence of a peak (maximum) at 1550 to 1600 $cm^{-1}$ in a spectrum obtained by laser Raman spectroanalysis at an excitation wavelength of 532 nm.

In this connection, "graphene-like" means a carbon hexagonal mesh structure of carbon atoms spread two-dimensionally by chemical bonds of sp2 hybrid orbital and some of carbon atoms composing the graphene-like structure may be substituted with other atoms such as nitrogen. Further, the above-mentioned graphene-like structure may be layered to form a graphite-like structure.

Further, the metal complex containing the macrocyclic ligand modified in the graphene-like state can be confirmed by measuring X-ray electron spectrometry (hereinafter, called as "XPS"). That is, in a spectrum obtained by XPS spectrum, the C1s peak showing the emission of photoelectrons of the 1s orbital of carbon atoms becomes a relatively wide peak and shows tailing in the high bonding energy side, existence of the graphene-like carbon can be confirmed as a qualitative index for the C1s peak and using this C1s peak as a qualitative index, the existence of the graphene-like carbon can be confirmed.

Further, in the case a nitrogen atom is contained as a coordinating atom in the macrocyclic ligand of the multinuclear metal complex before the treatment, in a spectrum obtained by XPS measurement, a peak is observed in N1s spectrum (396 eV to 404 eV) showing emission of photoelectron of 1s orbit of nitrogen atom. When XPS measurement is carried out for the metal complex using such a multinuclear metal complex, the N1s peak is often widened and thus the half width of the peak can be used as an index of the treatment.

The above-mentioned half width of the peak is preferably 2 eV or higher and it is presumed that such a metal complex contains multiple nitrogen atoms and modification is sufficiently promoted and therefore, it is preferable.

Particularly, in the case of comparison of the N1s spectra obtained by XPS measurement before and after treatment, if the half width of the peak of the metal complex becomes 1.3 times as wide as the half width of the multinuclear metal complex before treatment, the introduction quantity of nitrogen atom to the graphene-like structure is high and therefore, it is preferable. The alteration of the half width of the peak before and after is further preferably 1.4 times or higher and particularly more preferably 1.5 times or higher.

Next, another embodiment of the multinuclear complex of the invention will be described.

That is, another embodiment is a metal complex showing a ratio of weight loss by the treatment of 5 to 90 wt.% and a carbon content after the treatment of 5 wt.% or more in the case of subjecting a mixture of (a) a multinuclear metal complex and (b) a carbon carrier, an organic compound having a boiling point or melting point of 250°C or more or an organic compound having a thermal polymerization starting temperature of 250°C or less to any of heating treatment, radiation exposure treatment, and electric discharge treatment. Herein, the ratio of weight loss is on the basis of the total of (a) and (b) in the multinuclear metal complex mixture.

The mixing ratio of (a) and (b) in the multinuclear metal complex mixture is set on the basis of the metal atoms contained in the multinuclear metal complex (a). That is, the content of the metal atoms derived from (a) is set to be preferably 1 to 70 wt.% to the total weight of (a) and (b). The content of the metal atoms is more preferably 2 to 60 wt.% and even more preferably 3 to 50 wt.%.

Examples of the carbon carrier are carbon particles such as Norit, Ketjen black, Vulcan black, black pearl, acetylene black; fullerene of C60 and C70; carbon nanotubes, carbon nanohorns, carbon fibers and the like.

Examples of the organic compound having a boiling point or melting point of 250°C or more are aromatic carboxylic acid derivatives such as perylene-3,4,9,10-tetracarboxylic dianhydride, 3,4,9,10-perylenetetracarboxylic acid diimide, 1,4,5,8-naphthalenetetracarboxylic dianhydride, 1,4,5,8-naphthalenetetracarboxylic acid diimide, 1,4,5,8-naphthalenetetracarboxylic acid, 1,2,4,5-benzenetetracarboxylic acid (pyromellitic acid), and pyromellitic dianhydride. Herein, the boiling point or melting point can be measured by a conventionally known method and it may be selected from the measured values and also may be selected from the values disclosed in literatures or the like.

It may also be a calculated value computed by a calculation simulation or the like and it may be, for example,

selected from the calculated value of the boiling point or melting point registered in SciFinder, which is a computer software program provided by Chemical Abstract Service. In the compounds shown below, the remark "calc" in the boiling point (b.p.) is a calculation value registered in the above-mentioned SciFinder.

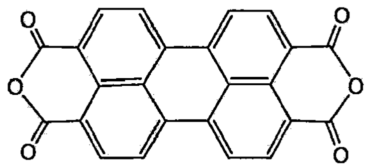

b.p.: 755 C° (calc)
m.p.: > 300 C°

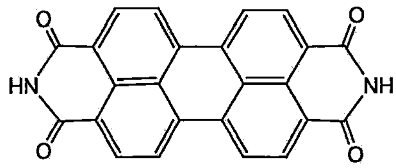

b.p.: 787 C° (calc)

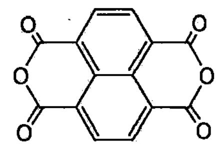

b.p.: 617 C° (calc)
m.p.: 450 C°

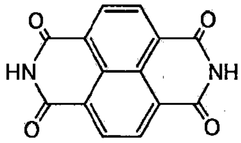

b.p.: 656 C° (calc)
m.p.: > 410 C°

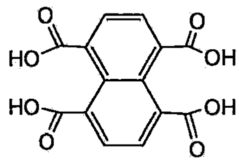

b.p.: 738 C° (calc)
m.p.: 180 C°

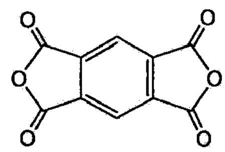

b.p.: 397-400 C°(calc)
m.p.: 238-286 C°

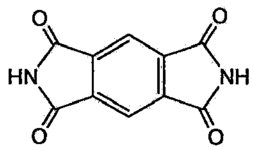

m.p.: 440 C°

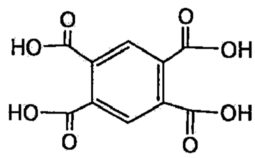

b.p.: 585 C° (calc)
m.p.: 272-276 C°

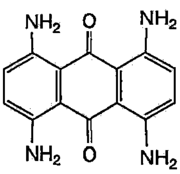

m.p.:332 C°
b.p.: 712 C°(calc)

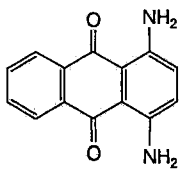

m.p.:269 C°
b.p.: 544 C°(calc)

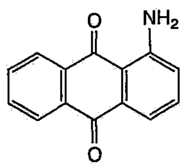

m.p.:254 C°
b.p.: 465 C°(calc)

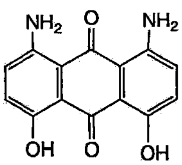

b.p.: 649 C°(calc)

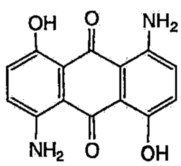

b.p.: 649 C°(calc)

Further, the compound having a thermal polymerization starting temperature of 250°C or less is an organic compound having an aromatic ring and further a double bond or a triple bond and examples are organic compounds

such as acenaphthylene and vinylnaphthylane. The numeral values attached to the respective compounds shown below are polymerization starting temperatures of the respective organic compounds. The numeral values are described in "Base of Carbonization Engineering" (1st edition, 2nd printing, Ohmsha, Ltd. 1982).

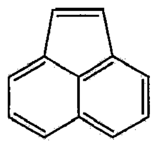

245 °C

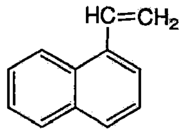

200 °C

The treatment condition at the time of treating such a multinuclear metal complex mixture is the same as the condition of treating the above-mentioned multinuclear metal complex.

The metal complex of the invention may be used in combination with various types of carriers and additives and the form can be processed depending on various applications. Examples of applications may be a decomposition catalyst of peroxides, an oxidation coupling catalyst for aromatic compounds, a catalyst for waste gas and wastewater purification, a redox catalyst layer for dye-sensitized solar cells, a carbon dioxide reduction catalyst, a catalyst for reforming hydrogen production, an oxygen sensor and the like.

Further, at the time of using the metal complex of the invention as a catalyst, it may be used in form of a composition containing a carbon carrier and/or a conductive polymer. It is advantageous in terms of improvement of the stability of the metal complex and improvement of catalytic activity. Examples of the conductive polymer are polyacetylene, polyaniline, polypyrrole and the like. Further, examples of the carbon carrier are same as those exemplified above. For the composition, more than one metal complexes of the invention may be used while being mixed and multiple carbon carriers or conductive polymers are also usable and carbon carriers and conductive polymers are used in combination.

Hereinafter, preferable applications of the metal complex of the invention will be described.

The metal complex of the invention is used preferably as a decomposition catalyst for peroxides and particularly preferably as a decomposition catalyst for hydrogen peroxide. In the case of use for a decomposition catalyst for hydrogen peroxide, the metal complex has a characteristic that it can decompose hydrogen peroxide into water and oxygen while suppressing hydroxy radical generation. Specifically, examples of applications are deterioration prevention agents for solid polymer electrolyte type fuel cells and ion conductive membranes for water electric decomposition, antioxidants for medical and agricultural agents and food products.

Further, it is also preferable as an oxidation coupling agent for aromatic compounds and in the case of this use, it can be used as a catalyst involving production of polymers such as polyphenylene ethers and polycarbonates. The manner for the use may be a method of directly adding the above-mentioned complex to a reaction solution, a method of depositing the complex on a zeolite, silica or the like.

Further, the metal complex of the invention may be used as a desulfurization and nitrogen oxide removal catalyst for converting sulfur oxide and nitrogen oxide contained in waste gases from various industrial plants and automobiles. A method of filling a tower with the metal complex to lead waste gases from plants into and a method of filling automotive mufflers with the metal complex can be exemplified.

Further, the metal complex of the invention can be used as a catalyst for converting CO in reformed hydrogen. CO or the like is contained in the reformed hydrogen and in the case of using reformed hydrogen for a fuel cell, it is a problem that a fuel electrode is poisoned with CO and therefore, it is desired to lower the concentration of CO as much as possible. With respect to a specific use manner, methods described in, for example, Chemical Communications, 2005, p3385 can be exemplified.

Further, in the case of using the metal complex of the invention as a carbon dioxide reduction catalyst, for example, $CO_2$ and $H_2$ may be reacted to produce $CH_4$ and CO. Specific use methods are same as the methods disclosed in Japanese Patent Application Laid-Open Nos. 7-68171 and 2002-104811.

Hereinafter, the invention will be described specifically based on Examples; however the invention should not be limited to Examples.

Synthesis Example 1 [Synthesis of multinuclear metal complex (A)]

The multinuclear metal complex (A) defined by the following reaction formula was synthesized according to a method described in Bulletin of Chemical Society of Japan, 68, 1105, (1995).

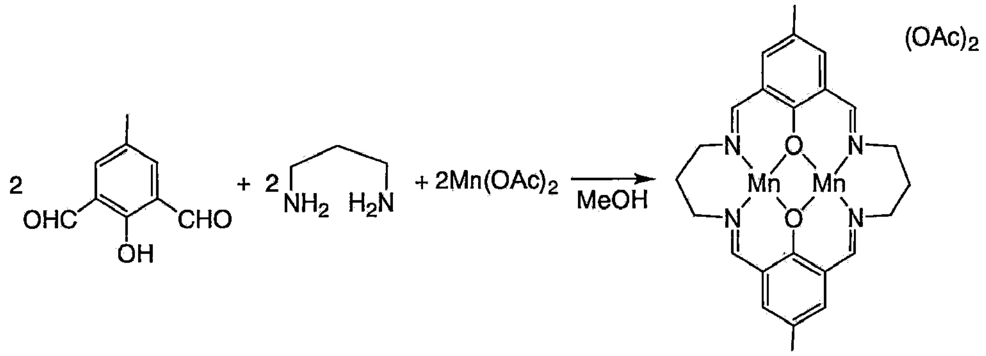


A 50 ml round-bottom flask was loaded with 10 ml of methanol containing 0.33 g of 4-methyl-2,6-diformylphenol and 0.49 g of manganese acetate tetrahydrate and the content was stirred at room temperature. Five ml of methanol containing 0.15 g of 1,3-propanediamine was gradually added to the obtained solution. After the above-mentioned mixture was stirred for 1 hour, yellow precipitate was produced. The precipitate was recovered by filtration and washed with methanol and thereafter vacuum dried to obtain a multinuclear metal complex (A) (produced amount 0.25 g: yield 39%). In the above-mentioned reaction formula "$(OAc)_2$" means two equivalents of acetate ions existed as a counter ion.

Elemental analysis value (%): Calcd. for $C_{28}H_{32}Mn_2N_4O_6$; C, 53.34; H, 5.12; N, 8,89. Found: C, 53.07; H, 5.12; N, 8,72.

Synthesis Example 2 [Synthesis of multinuclear metal complex (B)]

The multinuclear metal complex (B) defined by the following reaction formula was synthesized according to Australian Journal of Chemistry, 1970, 23, 2225.

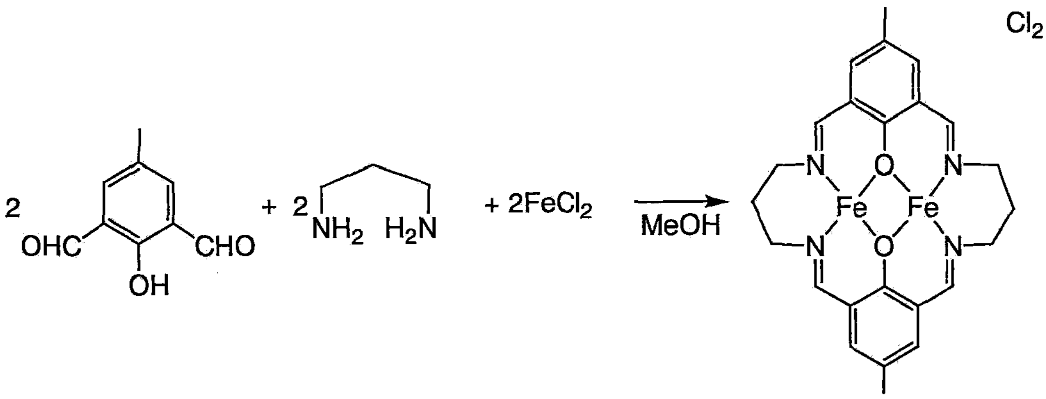


Under nitrogen atmosphere, a 50 ml round-bottom flask was loaded with 20 ml of a methanol solution containing 0.4 g of iron chloride tetrahydrate and 0.33 g of 4-methyl-2,6-diformylphenol and the solution was stirred at room temperature. Ten ml of methanol containing 0.15 g of 1,3-propanediamine was gradually added to the solution. After the above-mentioned mixture was stirred for 3 hours, red brown precipitate was produced. The precipitate was recovered by filtration and dried to obtain a multinuclear metal complex (B) (produced amount 0. 50 g: yield 85%). In the above-mentioned reaction formula the "$Cl_2$" means two equivalents of chloride ion existed as a counter ion.

Elemental analysis value (%): Calcd. for $C_{24}H_{26}Cl_2Fe_2N_4O_2$; C, 49.27; H, 4.48; N, 9.58. Found: C, 44.92; H, 4.94; N, 10.86.

Synthesis Example 3 [Synthesis of multinuclear metal complex (C)]

The multinuclear metal complex (C) defined by the following reaction formula was synthesized according to Australian Journal of Chemistry, 1970, 23, 2225.

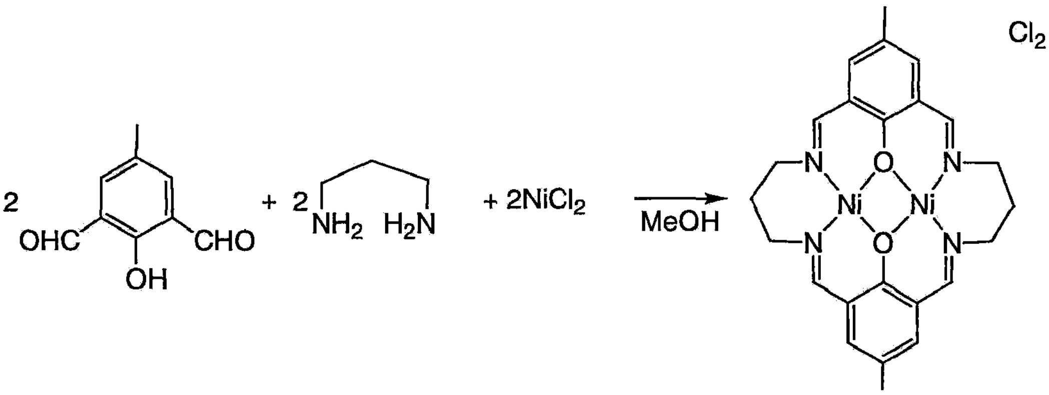


Under nitrogen atmosphere, a 100 ml round-bottom flask was loaded with 10 ml of an isopropanol solution containing 0.84 g of nickel chloride hexahydrate, 0.39 g of 1,3-propanediamine, and 0.58 g of 4-tert-butyl-2,6-diformylphenol and while being stirred, the solution was refluxed for 18 hours. On completion of the reaction, precipitate was washed with methanol and dried to obtain a multinuclear metal complex (C). In the above reaction formula, "$Cl_2$" means two equivalents of chloride ions existed as a counter ion.

Elemental analysis value (%): Calcd. for $C_{30}H_{38}Cl_2Fe_2N_4O_2$; C, 53.84; H, 5.72; N, 8.37. Found: C, 53.32; H, 5.74; N, 8.25.

Synthesis Example 4 [Synthesis of multinuclear metal complex (D)]

The multinuclear metal complex (C) defined by the following reaction formula was synthesized according to the method described in Chemische Berichte 1994, 127, 465.

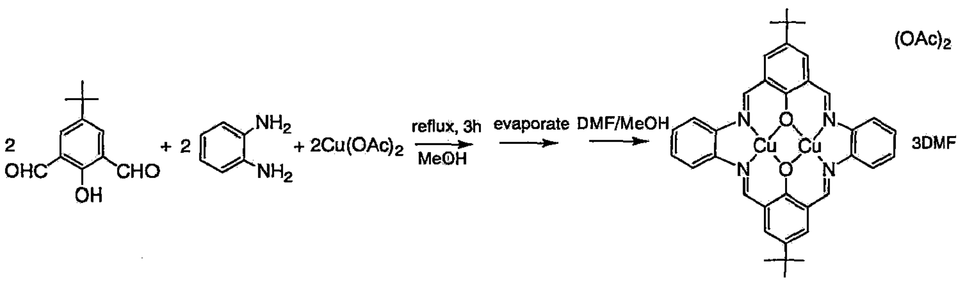


While being stirred 1 ml of a methanol solution containing 0.1g of 1, 2-phenylenediamine was added to a 25 ml round-bottom flask containing 2.5 ml of a methanol solution containing 0.20 g of copper acetate monohydrate. Successively, 2 ml of methanol containing 0.21 g of 4-tert-butyl-2,6-diformylphenol was gradually added and thereafter, refluxing was carried out for 3 hours. After the solvent was removed by an evaporator, the residue was dissolved in a small amount of N,N-dimethylfomamide and after 10 ml of methanol was added and the mixture was cooled in a refrigerator to produce a brown precipitate. The precipitate was recovered by filtration and dried to obtain a multinuclear metal complex (D) (produced amount 0.16 g: yield 31%). In the above-mentioned reaction formula "$(OAc)_2$" means two equivalents of acetate ions existed as a counter ion.

Elemental analysis value (%): Calcd. for $C_{49}H_{61}Cu_2N_7O_9$; C, 57.75; H, 6.03; N, 9.62. Found: C, 55.12; H, 5.42; N, 10.22.

Synthesis Example 5 [Synthesis of multinuclear metal complex (E)]

Themultinuclearmetal complex (E) definedbythefollowing reaction formulawas synthesized according to Australian Journal of Chemistry, 1970, 23, 2225.

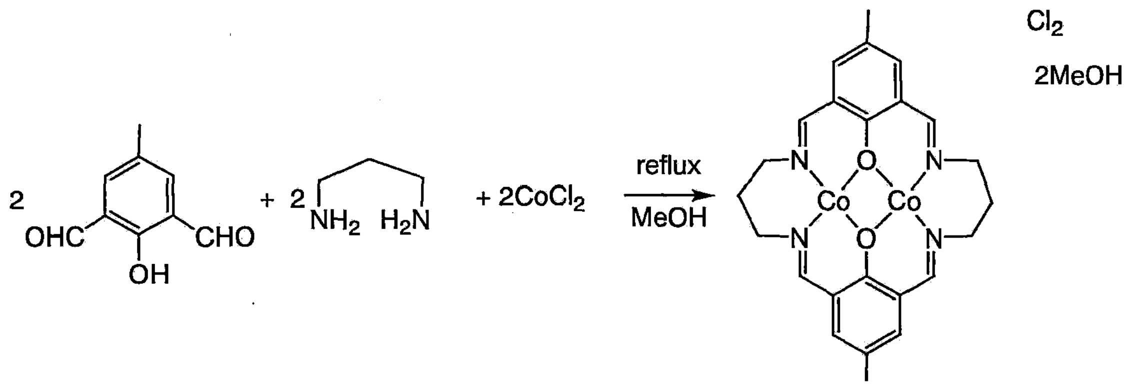


Under nitrogen atmosphere, a 100 ml round-bottom flask was loaded with 50 ml of a methanol solution containing 1.9 g of cobalt chloride hexahydrate and 1.31 g of 4-methyl-2,6-diformylphenol and the solution was stirred at room temperature. Twenty ml of methanol containing 0.59 g of 1,3-propanediamine was gradually added. The mixture was refluxed for 3 hours to produce dark brown precipitate. The precipitate was recovered by filtration and dried to obtain a multinuclear metal complex (E) (produced amount 1. 7 5 g: yield 74%). In the above reaction formula, "$Cl_2$" means two equivalents of chloride ion existed as a counter ion and "2MeOH" means two equivalents of methanol molecules existed as other ligands.

Elemental analysis value (%): Calcd. for $C_{26}H_{34}Cl_2Co_2N_4O_4$; C, 47.65; H, 5.23; N, 8.55. Found: C, 46.64; H, 5.02; N, 8.58.

Synthesis Example 6 [Synthesis of multinuclear metal complex (F)]

Themultinuclearmetal complex (F) definedbythefollowing reaction formula was produced by the following method.

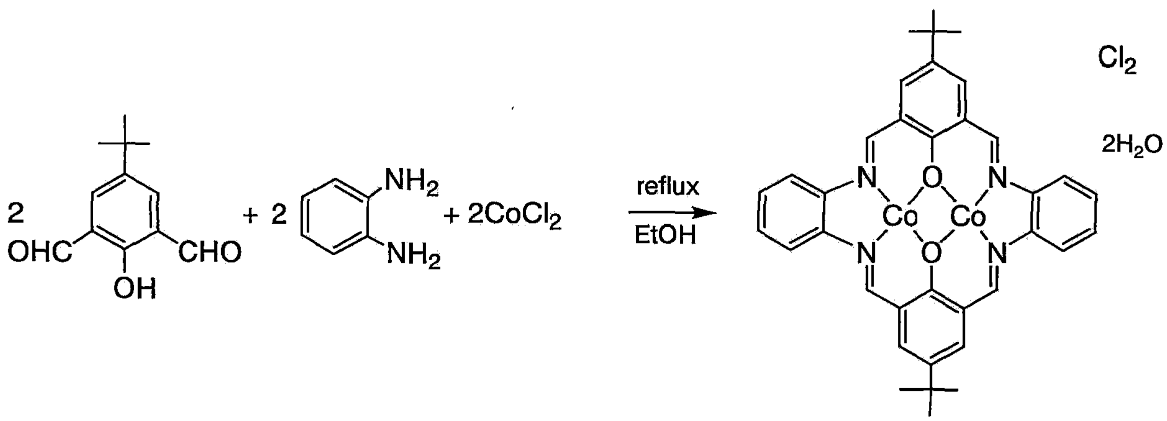


Under nitrogen atmosphere, a 50 ml round-bottom flask was loaded with 10 ml of a ethanol solution containing 0.476 g of cobalt chloride hexahydrate and 0.412 g of 4-tert-butyl-2,6-diformylphenol and the solution was stirred at room temperature. Five ml of ethanol containing 0.216 g of o-phenylenediamine was gradually added. The mixture was refluxed for 2 hours to produce dark brown precipitate. The precipitate was recovered by filtration and dried to obtain a

multinuclear metal complex (F) (produced amount 0.465 g: yield 63%). In the above reaction formula, "$Cl_2$" means two equivalents of chloride ions existed as a counter ion and "$2H_2O$" means two equivalents of water molecules existed as other ligands.

Elemental analysis value (%): Calcd. for $C_{36}H_{38}Cl_2Co_2N_4O_4$; C, 55.47; H, 4.91; N, 7.19. Found: C, 56.34; H, 4.83; N, 7.23.

Synthesis Example 7 [Synthesis of multinuclear metal complex (G)]

The multinuclear metal complex (G) defined by the following reaction formula was produced by the following method.

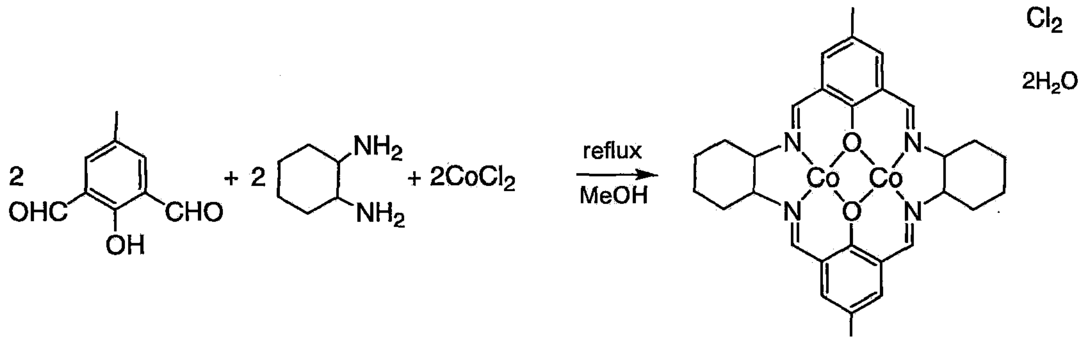


Under nitrogen atmosphere, a 50 ml round-bottom flask was loaded with 10 ml of a methanol solution containing 0.476 g of cobalt chloride hexahydrate and 0.328 g of 4-methyl-2,6-diformylphenol and the solution was stirred at room temperature. Five ml of a methanol solution containing 0.228 g of cyclohexyldiamine was gradually added. The mixture was refluxed for 2 hours to produce dark brown precipitate. The precipitate was recovered by filtration and dried to obtain a multinuclear metal complex (G) (produced amount 0.141 g: yield 21%). In the above reaction formula, "$Cl_2$" means two equivalents of chloride ions existed as a counter ion and "$2H_2O$" means two equivalents of water molecules existed as other ligands.

Elemental analysis value (%): Calcd. for $C_{30}H_{38}Cl_2Co_2N_4O_4$; C, 50.93; H, 5.41; N, 7.92. Found: C, 49.60; H, 5.47; N, 8.04.

Synthesis Example 8 [Synthesis of multinuclear metal complex (H)]

Themultinuclearmetalcomplex (H) definedbythefollowing reaction formula was produce by a method described in Journal of Chemical Society, Dalton Transactions, 1996, 1223.

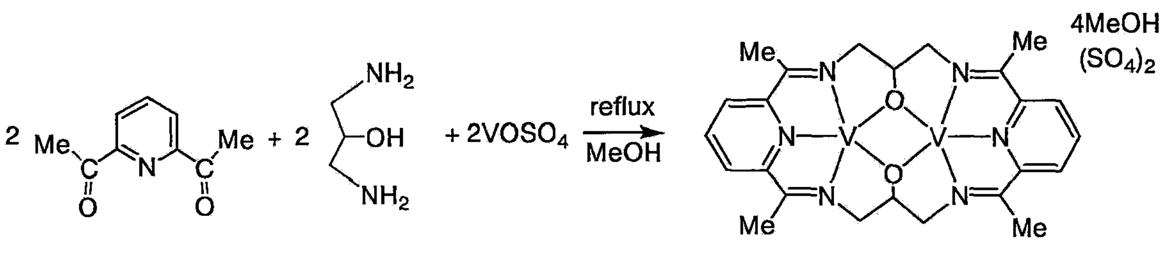


Under nitrogen atmosphere, a 100 ml round-bottom flask was loaded with 30 ml of a methanol solution containing 1.63 g of vanadium oxysulfate and 1.63 g of 2,6-diacetylpyridine and the solution was heated at 80°C while being stirred. Twenty ml of a methanol solution containing 0.90 g of 1,3-diaminopropane was dropwise added over 30 minutes. The mixture was refluxed for 8 hours to produce dark bluish-purple precipitate. The precipitate was recovered by filtration and dried to obtain a multinuclear metal complex (H) (produced amount 3.1 g: yield 45%). In the above reaction formula,

"$(SO_4)_2$" means two equivalents of sulfate ions existed as a counter ion and "4MeOH" means four equivalents of water molecules existed as other ligands.

Elemental analysis value (%): Calcd. for $C_{28}H_{44}V_2N_6O_{14}S_2$; C, 39.35; H, 5.19; N, 9.83. Found: C, 39.73; H, 5.44; N, 10.42.

Examples 1 to 8 [Heating treatment of multinuclear metal complexes]

Using a thermogravitational/differential thermal analyzer (EXSTAR-6300, manufactured by Seiko Instruments Inc., hereinafter, called as thermal analyzer), the weight alteration (TGA) at the time of heat treatment was measured for the multinuclearmetal complexes (A) to (H) obtained in the Synthesis Examples 1 to 8. The measurement conditions were under nitrogen atmosphere at temperature increase speed of 10°C/min and a platinum plate or an aluminum plate was used for the heat treatment.

The analysis results (analysis charts) of the multinuclear metal complexes (A) to (H) are shown respectively in Figs. 1 to 8.

Based on the findings obtained by thermogravitational analysis results, firing treatment was carried out in a manner that the ratio of weight loss at the time of heat treatment became 5 wt.%. That is, each of the multinuclear metal complexes was subjected to two-hour heat treatment at an aimed temperature under nitrogen atmosphere using a tubular furnace.

The tubular furnace used for the heat treatment and heat treatment conditions are shown below.

Tubular furnace: EPKRO-14R, program-controllable opening and closing type tubular furnace, manufactured by Isuzu Seisakusho

Heat treatment atmosphere: nitrogen gas flow 200 ml/min

Temperature increase speed and temperature decrease speed: 200°C/h

Table 1 shows the used multinuclear metal complexes, heat treatment temperature, and ratio of weight loss after the treatment. Further, the carbon content (elemental analysis value) after the heat treatment is also shown together with the carbon content before heat treatment.

Table 1

| Example | Used multinuclear metal complex | Heat treatment temperature | Wt. loss ratio (wt.%) | Carbon content (wt.%) |
|---|---|---|---|---|
| 1 | Multinuclear metal complex (A) | Before heat treatment | - | 53.07 |
| | | 500˚C Heat treatment | 29 | 48.42 |
| 2 | Multinuclear metal complex (B) | Before Heat treatment | - | 44.92 |
| | | 500°C Heat treatment | 30 | 50.88 |
| 3 | Multinuclear metal complex (C) | Before heat treatment | - | 42.32 |
| | | 500°C Heat treatment | 42 | 55.41 |
| 4 | Multinuclear metal complex (D) | Before heat treatment | - | 55.12 |
| | | 500°C Heat treatment | 30 | 50.88 |
| 5 | Multinuclear metal complex (E) | Before heat treatment | - | 46.64 |
| | | 500°C Heat treatment | 37 | 49.02 |
| 6 | Multinuclear metal complex (F) | Before heat treatment | - | 56.34 |
| | | 500°C Heat treatment | 30 | 59.11 |
| 7 | Multinuclear metal complex (G) | Before heat treatment | - | 56.34 |
| | | 450°C Heat treatment | 32 | 51.22 |
| 8 | Multinuclear metal complex (H) | Before heat treatment | - | 39.73 |
| | | 500°C Heat treatment | 7.5 | 41.25 |

Herein, the complexes obtained by heating the multinuclear metal complexes (A) to (H) are named as modified complexes (A) to (H), respectively.

Example 9 [Laser Raman spectrum measurement for multinuclear metal complex (A) and modified complex (A)]

Fig. 9 shows the laser Raman spectra of multinuclear metal complex (A) and modified complex (A). The measurement was carried out in the following conditions.

Apparatus used: microscopic laser Raman spectrometer NSR 1000 (JASCO Corporation)
Excitation wavelength: 532 nm
Objective lens: 50 magnification
Measurement range: 200 to 3900 $cm^{-1}$
From Fig. 9, it can be found that the modified complex (A) has a maximum peak at 1580 $cm^{-1}$. Accordingly, it is implied that graphene-like carbon was produced by the treatment.

Example 10 [X-ray photoelectron spectrum measurement for multinuclear metal complex (E) and modified complex (E)]

X-ray photoelectron spectra of multinuclear metal complex (E) and modified complex (E) were measured. For the X-ray photoelectron spectroscopy (XPS), SSX-100 manufactured by SII was used. A monochromatic AlKa ray (1486.6 eV, X-ray spot 1000 μm) was employed as X-ray. Further, for neutralizing the electric charge during the measurement, a Ni mesh was put on a sample and a neutral electron gun was used for the measurement. The bonding energy of the spectra was calibrated as the C-C and C-H bonds of C1s had a bonding energy of 284.6 eV.
Fig. 10 shows the C1s spectra and Fig. 11 shows the N1s spectra. In Fig. 10, the peak of C1s of the modified complex (E) showed tailing in the higher bonding energy side and it implies production of the graphene-like carbon.
Further, in Fig. 11, the half width of the peak of the modified complex (E) was 3.5 eV. The half width of the peak of the modified complex (E) was 2.3 times as wide as the half width of the peak of the multinuclear metal complex (E).

Examples 11 to 18 and Comparative Examples 1 to 8 [Evaluation tests of metal retaining capability of modified complex (A) to modified complex (H)]

Each of the modified complex (A) to modified complex (H) obtained in Examples 1 to 8 in an amount of 10 mg was immersed in 2 ml of a tartaric acid buffer solution (produced from 0.2 mol/L of aqueous tartaric acid solution and 0.1 mol/L of aqueous sodium tartarate solution) at pH 4 and stirred at room temperature for 20 minutes. After the solution was filtered, the metal amount contained in the filtrate was quantitatively analyzed by inductively-coupled plasma atomic emission spectrometry (ICP-AES) or absorption photometry to calculate the metal retention ratio.
Further, the multinuclear metal complex (A) to multinu clear metal complex (H) which were not subjected to heat tre atment were also subjected to the evaluation test for the me tal retention capability and the results were compared with the results of Examples 11 to 18. The same operation was car ried out and the metal amounts contained in the filtrates we re measured in the same manner as complexes (A) to (H) in Exa mples 11 to 18 to calculate the metal retention ratios, exce pt that the complexes (A) to (H) in Examples 11 to 18 were ch anged to be the multinuclear metal complexes (A) to (H). Th e multinuclear metal complexes (A) to (H) which were not sub jected to the heat treatment were found inferior in the meta 1 retaining capability as compared with the respective modif ied complexes (A) to (H).

Table 2

| - | Evaluation sample | Metal retention ratio |
|---|---|---|
| Example 11 | Modified complex (A) | 91% |
| Example 12 | Modified complex (B) | 99% |
| Example 13 | Modified complex (C) | 99% |
| Example 14 | Modified complex (D) | 88% |
| Example 15 | Modified complex (E) | 76% |
| Example 16 | Modified complex (F) | 94% |
| Example 17 | Modified complex (G) | 93% |
| Example 18 | Modified complex (H) | 93% |
| Comparative Example 1 | Multinuclear metal complex (A) | 18% |
| Comparative Example 2 | Multinuclear metal complex (B) | 17% |

(continued)

| - | Evaluation sample | Metal retention ratio |
|---|---|---|
| Comparative Example 3 | Multinuclear metal complex (C) | 24% |
| Comparative Example 4 | Multinuclear metal complex (D) | 84% |
| Comparative Example 5 | Multinuclear metal complex (E) | 9% |
| Comparative Example 6 | Multinuclear metal complex (F) | 73% |
| Comparative Example 7 | Multinuclear metal complex (G) | 12% |
| Comparative Example 8 | Multinuclear metal complex (H) | 38% |

Example 19 [Hydrogen peroxide decomposition test for modified complex (A)]

A two-neck flask was loaded with 1.6 mg (about 8 μmol (per 1 metal atom) of the modified complex (A) and a tartaric acid/sodium tartarate buffer solution (1.00 ml (produced from an aqueous 0.20 mol/L tartaric acid solution and an aqueous 0.10 mol/L sodium tartarate solution) and ethylene glycol (1.00 ml) were added as solvents to the flask. The obtained solution was used as a catalyst-mixed solution.

A septum was attached to one side of the two-neck flask containing the catalyst-mixed solution and the other side was joined to a gas burette. After the flask was shaken at 80°C for 5 minutes, an aqueous hydrogen peroxide solution (11.4 mol/L, 0.20 ml (2.28 mmol)) was added with a syringe and hydrogen peroxide decomposition reaction was carried out at 80°C for 20 minutes. The generated oxygen was measured with gas burette to quantitatively measure the decomposed hydrogen peroxide.

The decomposed hydrogen peroxide amount was calculated from the oxygen volume generated in the hydrogen peroxide decomposition test. The generated gas volume value v by actual measurement was converted into the following gas volume V in consideration of the steam pressure at 0°C and 101325 Pa (760 mmHg).

The result is shown in Fig. 12. It was confirmed that the modified complex (A) of the invention had high generated gas volume as compared with that in the blank test described below and thus had a catalytic effect on hydrogen peroxide decomposition.

$$V = \frac{273v(P-p)}{760(273+t))}$$

(In the formula, P: atmospheric pressure (mmHg), p: vapor pressure of water (mmHg), t: temperature (°C), v: actually measured generated gas volume (ml), V: gas volume (ml) at 0°C and 101325 Pa (760 mmHg)).

[Blank test]

A two-neck flask was loaded with 1.00 ml of a tartaric acid/sodium tartarate buffer solution (produced from an aqueous 0.20 mol/L tartaric acid solution and an aqueous 0.10 mol/L sodium tartarate solution, pH 4.0) and 1.00 ml of ethylene glycol as solvents. A septum was attached to one side of the two-neck flask and the other side was joined to a gas burette. After the flask was shaken at 80°C for 5 minutes, an aqueous hydrogen peroxide solution (11.4 mol/L, 0.20 ml (2.28 mmol)) was added and the generated gas was quantitatively measured with gas burette.

It was presumed that air dissolved in the solution could be detected in this blank test.

Comparative Example 9 [Hydrogen peroxide decomposition test for multinuclear metal complex (A)]

The same test as that in Example 19 was carried out except that the modified complex (A) in Example 19 was changed to the multinuclear metal complex (A). The result is shown together with result of Example 19 in Fig. 12.

The gas generation amount was not different from that of the blank test and the catalyst effect on the hydrogen peroxide decomposition was not confirmed.

Example 2 0 [Occurrence of radical generation in hydrogen peroxide decomposition test by complex (A)]

A two-neck flask was loaded with a complex (A) (1.6 to 2.2 mg, about 8 μm mol (per one metal atom)) and 21.1 mg of poly(4-stylenesulfonic acid) sodium salt (weight average molecular weight: about 70,000, Aldrich commercialized product) and a tartaric acid/sodium tartarate buffer solution (1.00 ml, produced from an aqueous 0.20 mol/L tartaric acid solution and an aqueous 0.10 mol/L sodium tartarate solution, pH 4.0) and 1.00 ml of ethylene glycol as solvents were added there. The obtained solution was used as a catalyst-mixed solution.
A septum was attached to one side of the two-neck flask containing the catalyst-mixed solution and the other side was joined to a gas burette. After the flask was shaken at 80°C for 5 minutes, an aqueous hydrogen peroxide solution (11.4 mol/L, 0.20 ml (2.28 mmol)) was added with a syringe and hydrogen peroxide decomposition reaction was carried out at 80°C for 20 minutes. The generated oxygen was measured with gas burette to quantitatively measure the decomposed hydrogen peroxide. Thereafter, the reaction solution was diluted with a water/acetonitrile mixed solution (water : acetonitrile = 7 : 3 (v/v)) to adjust the solution volume to be 10.0 ml and the resulting solution was filtered with a syringe filter. The filtrate was analyzed by GPC and the GPC pattern of the poly(sodium 4-styrenesulfonate) after the test was obtained by the following method and compared with that for the poly(4-styrenesulfonic acid) sodium salt.
GPC patterns are shown in Fig. 13.
Lowering of molecular weight was scarcely observed in the peaks of poly(4-styrenesulfonic acid) and thus it was confirmed that no radical specie such as hydroxy radical capable of decomposing poly(4-styrenesulfonic acid) was generated at the time of decomposing the hydrogen peroxide by the multinuclear metal complex (A). In consideration of the case of hydrogen peroxide decomposition by a mononuclear metal complex in which radical specie generation is frequently caused, the result of this test proves that the complex (A) of the invention retained a multinuclear structure.
[GPC analysis conditions]
Column: TSKgel a-M, manufactured by Tosoh Corporation (13 μm, 7.8 mmφ ×30 cm)
Column temperature: 40°C
Mobile phase: 50 mmol/L aqueous ammonium acetate; $CH_3CN$ = 7 : 3 (v/v)
Flow velocity: 0.6 mL/min
Detector: RI
Injection amount: 50 μL

Example 21 [Heat treatment of mixture of multinuclear metal complex (A)]

A mixture of 0.053 g of the multinuclear metal complex (A) obtained in Synthesis Example 1 and 0.20 g of perylene-3,4,9,10-tetracarboxylic acid dianhydride was evenly mixed using an agate mortar. The mixture was subjected to heating treatment to 500°C (temperature increase speed 10°C/min) in a thermoanalyzer. The ratio of weight loss after the treatment was 28% to the initial weight of the entire mixture. The carbon content in the treated product was 57% as the lower limit by calculation of the ratio of weight loss and the coexisting element ratio.

Example 22 [Heat treatment of mixture of multinuclear metal complex (E)]

A mixture of 20 mg of the multinuclear metal complex (E) obtained in Synthesis Example 5 and 160 mg of Ketjen Black (EC 300 J, Lion Corp.) was mixed with 15 ml of ethanol and stirred at room temperature for 20 minutes to obtain a slurry. The slurry was dried at room temperature under reduced pressure of 1.5 Torr for 12 hours. Using a tubular furnace, the dried product was subjected to heating treatment at 450°C in 200 mL/min nitrogen current for 2 hours. The ratio of weight loss after the treatment was 5.3% to the initial weight of the entire mixture. The carbon content in the treated product was 89.56%.

Example 23 [Heat treatment of mixture of multinuclear metal complex (F)]

A mixture of 20 mg of the multinuclear metal complex (F) obtained in Synthesis Example 6 and 160 mg of Ketjen Black (EC 300 J, Lion Corp.) was mixed with 15 ml of ethanol and stirred at room temperature for 20 minutes to obtain a slurry. The slurry was dried at room temperature under reduced pressure of 1.5 Torr for 12 hours. Using a tubular furnace, the dried product was subjected to heating treatment at 500°C in 200 mL/min nitrogen current for 2 hours. The ratio of weight loss after the treatment was 7.2% to the initial weight of the entire mixture. The carbon content in the treated product was 85.85%.
According to the invention, a catalyst having high reaction activity as a catalyst for peroxide decomposition reaction, oxide decomposition reaction, oxygen addition reaction, oxidative coupling reaction, dehydrogenation reaction, hydrogenation reaction, and redox reaction involving electron transfer such as electrode reaction and the like can be obtained and the catalyst is preferably usable for synthesis of organic compounds and polymer compounds, additives,

reforming agents, batteries, and materials for sensors and thus is remarkably advantageous for industrial applications.

## Claims

1. A multinuclear metal complex having at least one macrocyclic ligand having 5 to 15 coordinating atoms and two or more metal atoms, wherein the metal complex is subjected to any of heating treatment, radiation exposure treatment, and electric discharge treatment, thereby the complex shows a ratio of weight loss by the treatment of 5 to 90 wt.% and a carbon content after the treatment of 5 wt.% or more.

2. The metal complex according to claim 1, wherein the metal atoms are transition metal atoms belonging to Group 4 to Group 6 in the periodic table.

3. The metal complex according to claim 1 or 2, wherein the number of the metal atoms is 2 to 10.

4. The metal complex according to claim 1, wherein the coordinating atoms are selected from a group consisting of a carbon atom, a nitrogen atom, an oxygen atom, a phosphorus atom, and a sulfur atom.

5. The metal complex according to claim 1, wherein the macrocyclic ligand is a macrocyclic ligand defined by the following formula (I)

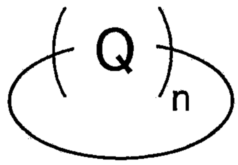

(I)

wherein Q denotes a divalent organic group containing one or more of coordinating atoms selected from a group consisting of a carbon atom, a nitrogen atom, an oxygen atom, a phosphorus atom, and a sulfur atom and n denotes an integer of 5 or more and 15 or less.

6. The metal complex according to claim 1, wherein the treatment is a treatment at a temperature of 250°C or more and 1000°C or less.

7. The metal complex according to claim 1, wherein the ratio of the weight loss by the treatment is 7 wt.% or more and 90 wt.% or less.

8. The metal complex according to claim 1, wherein the carbon content of the multinuclear metal complex is 10 wt.% or more.

9. The metal complex according to claim 1, wherein the metal complex has a peak in a range of 1550 to 1600 $cm^{-1}$ in a spectrum measured by laser Raman spectrometry at an excitation wavelength of 532 nm.

10. The metal complex according to claim 1, wherein the half width of a signal of the metal complex having the maximum peak in a range of 396 eV to 404 eV in an N1s spectrum by X-ray photoelectric spectroscopy is 2 eV or more.

11. The metal complex according to claim 10, wherein the half width of the signal of the metal complex having the maximum peak in a range of 396 eV to 404 eV in an N1s spectrum by X-ray photoelectric spectroscopy is 1.3 times as large as the half width of the signal of the multinuclear metal complex before the treatment.

12. A metal complex showing a ratio of weight loss by the treatment of 5 to 90 wt.% and a carbon content after the treatment of 5 wt.% or more in the case of subjecting a mixture of (a) a multinuclear metal complex having at least one macrocyclic ligand having 5 to 15 coordinating atoms and two or more metal atoms and (b) a carbon carrier or at least one organic compound selected from organic compounds having a boiling point or melting point of 250°C or more and organic compounds having a thermal polymerization starting temperature of 250°C or less to any of heating treatment, radiation exposure treatment, and electric discharge treatment.

**13.** A composition containing the multinuclear metal complex according to one of claims 1 to 12 as well as a carbon carrier and/or a conductive polymer.

**14.** A catalyst containing the multinuclear metal complex according to one of claims 1 to 12 and/or the composition according to claim 13.

Fig. 1

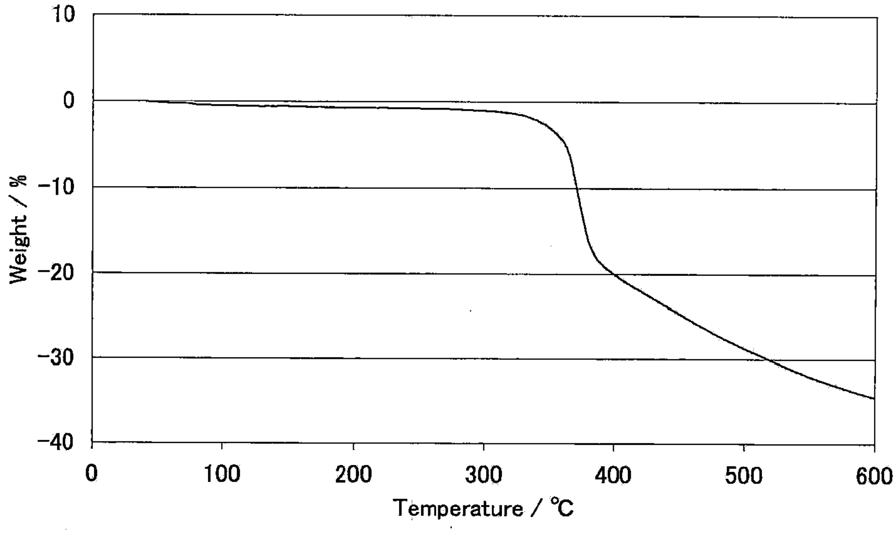
10
0
-10
-20
-30
-40
Weight / %
0
100
200
300
400
500
600
Temperature / ℃

Fig. 2

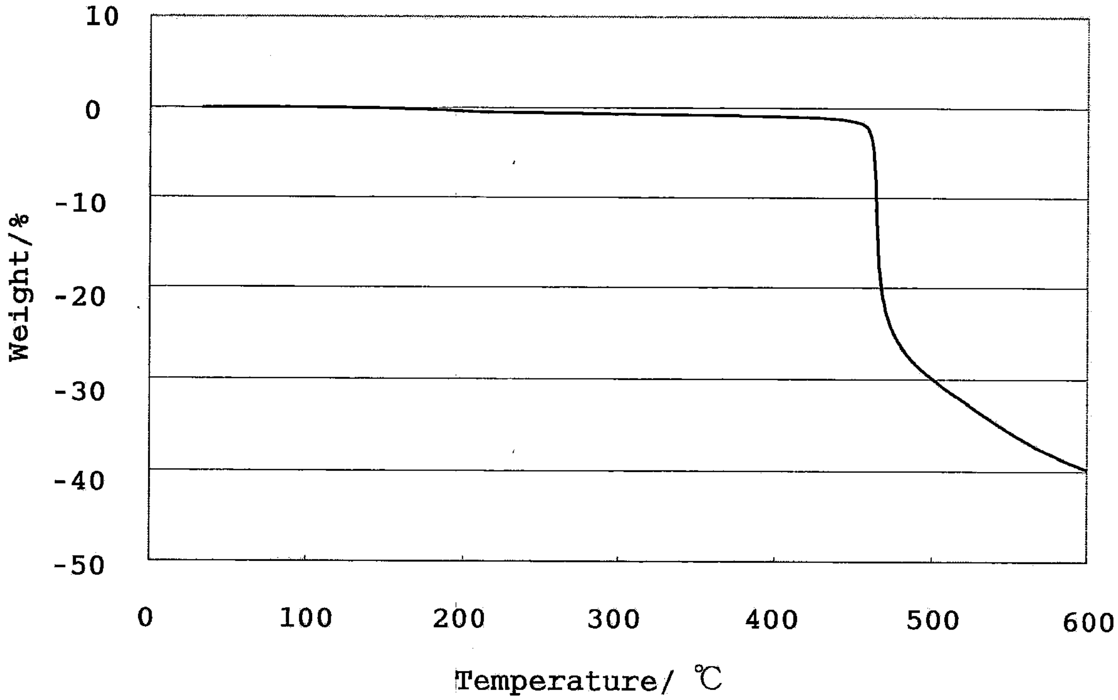
10
0
-10
-20
-30
-40
-50
Weight/%
0
100
200
300
400
500
600
Temperature/ ℃

Fig. 3

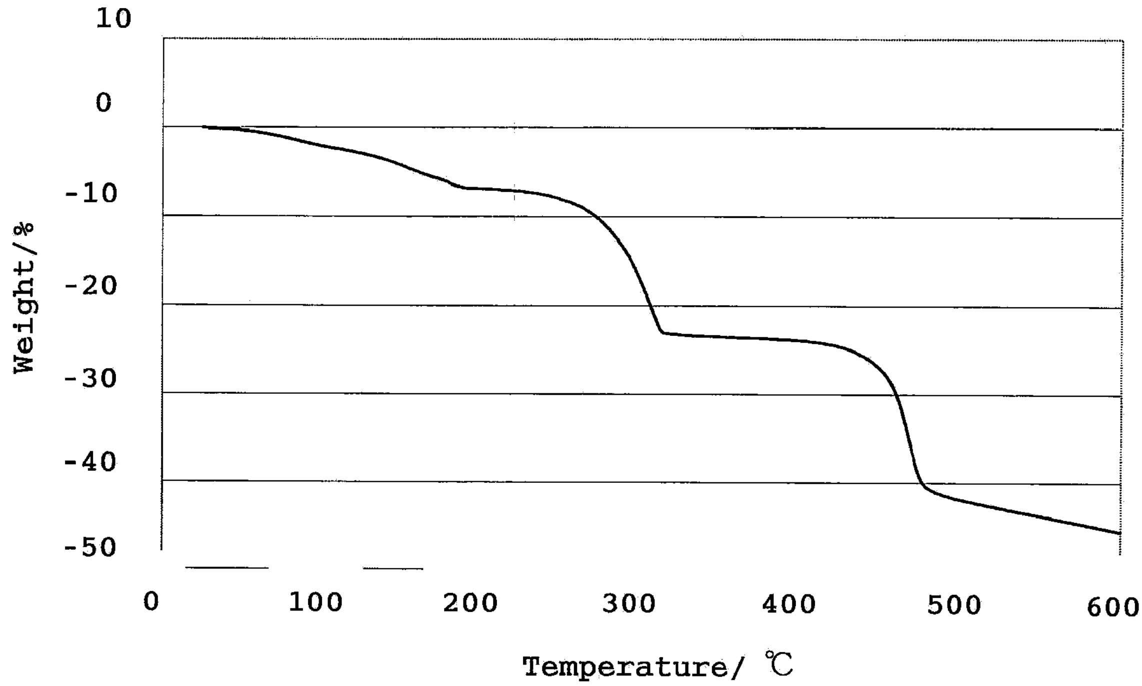
10
0
-10
-20
-30
-40
-50
Weight/%
0
100
200
300
400
500
600
Temperature/ ℃

Fig. 4

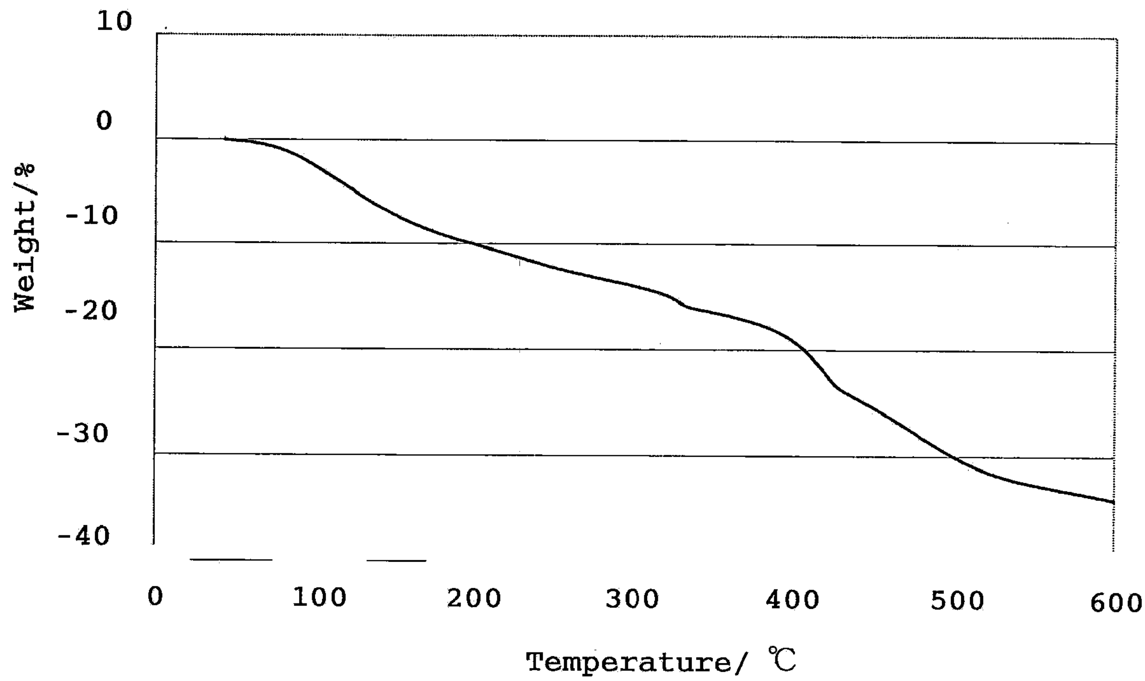
10
0
-10
-20
-30
-40
Weight/%
0
100
200
300
400
500
600
Temperature/ ℃

Fig. 5

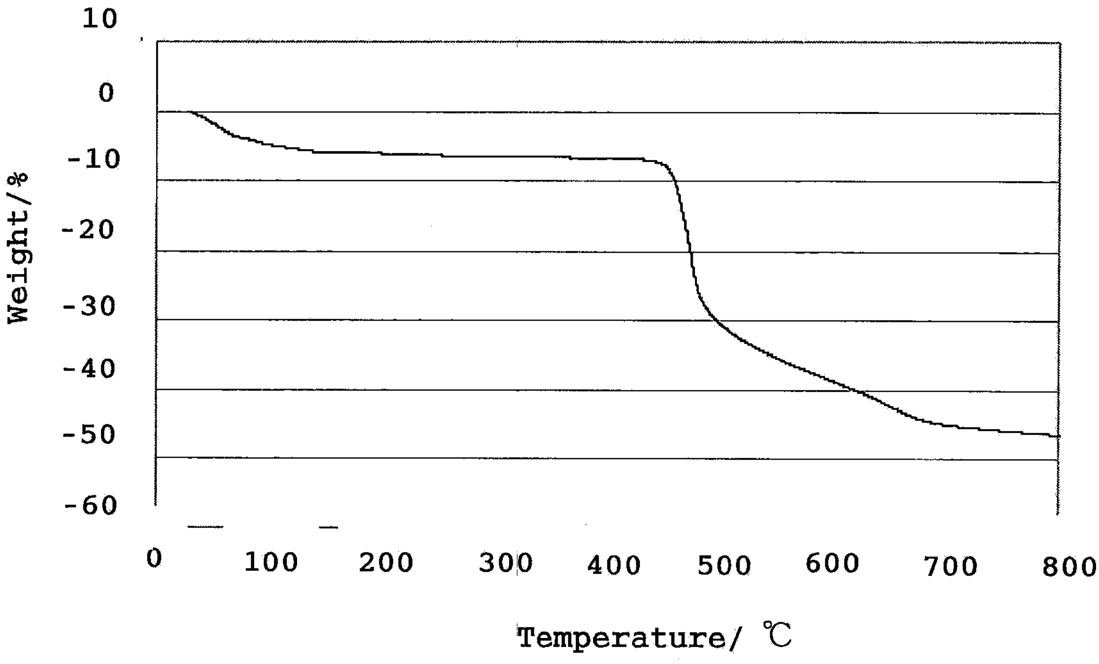
10
0
-10
-20
-30
-40
-50
-60
Weight/%
0
100
200
300
400
500
600
700
800
Temperature/ ℃

Fig. 6

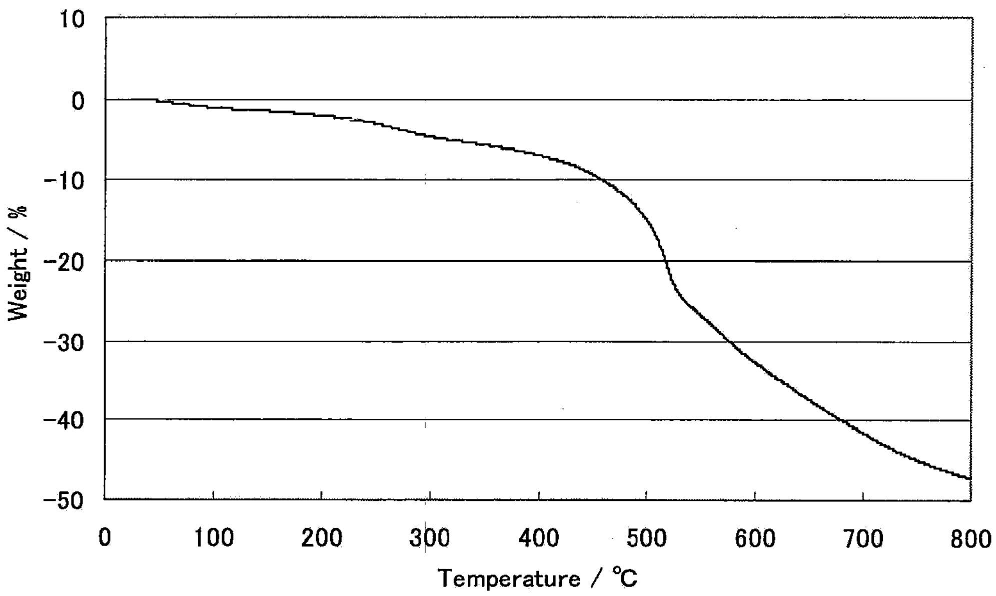
10
0
-10
-20
-30
-40
-50
Weight / %
0
100
200
300
400
500
600
700
800
Temperature / ℃

Fig. 7

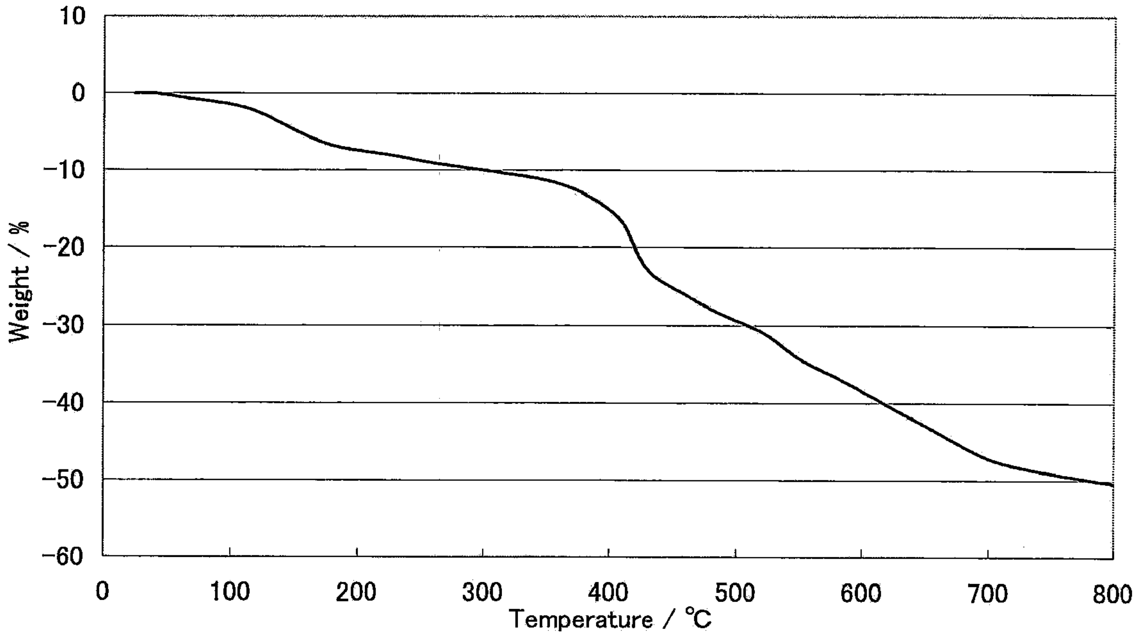
10
0
-10
-20
-30
-40
-50
-60
Weight / %
0
100
200
300
400
500
600
700
800
Temperature / ℃

Fig. 8

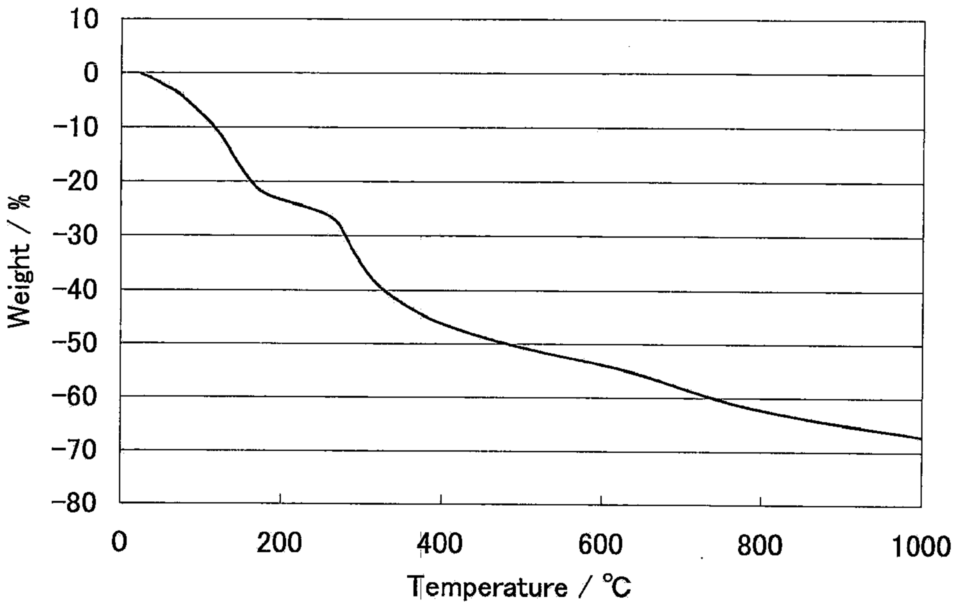
10
0
-10
-20
-30
-40
-50
-60
-70
-80
Weight / %
0
200
400
600
800
1000
Temperature / °C

Fig.9

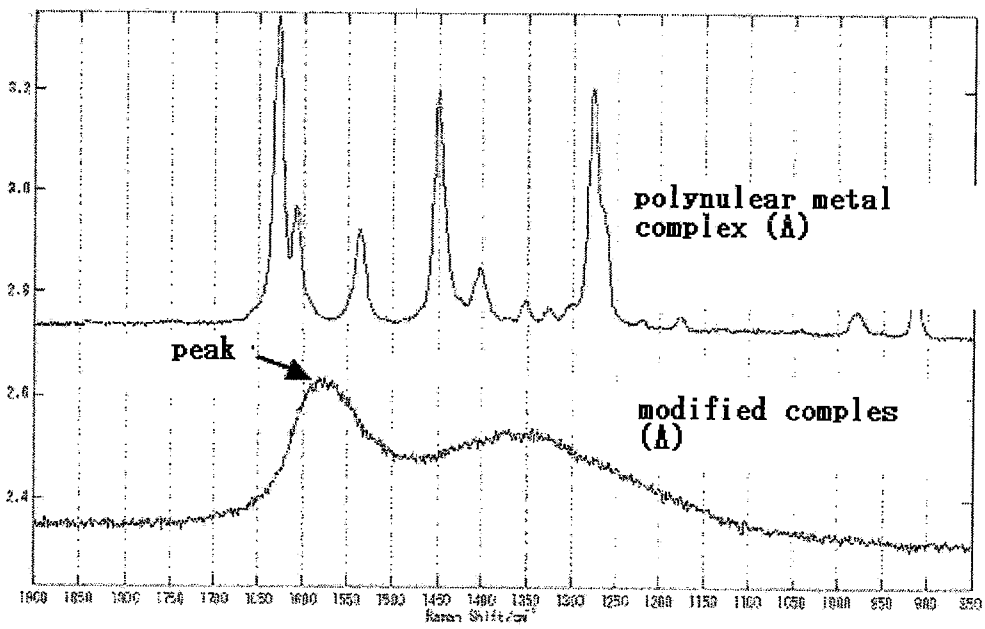
polynulear metal complex (A)
peak
modified comples (A)

Fig. 1 0

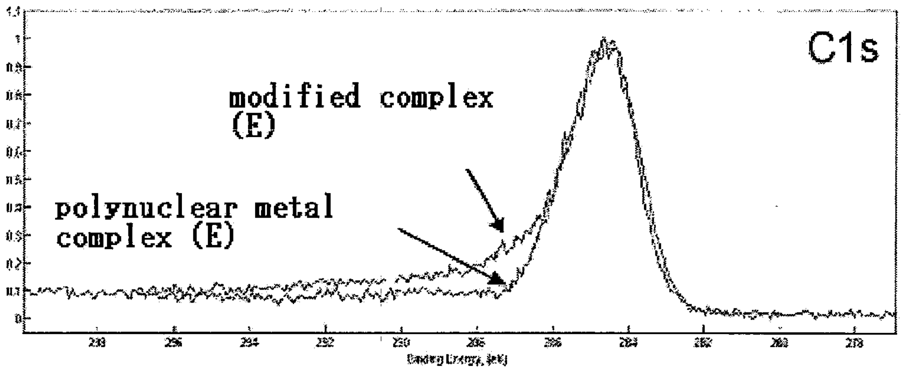
C1s
modified complex
(E)
polynuclear metal
complex (E)

Fig. 1 1

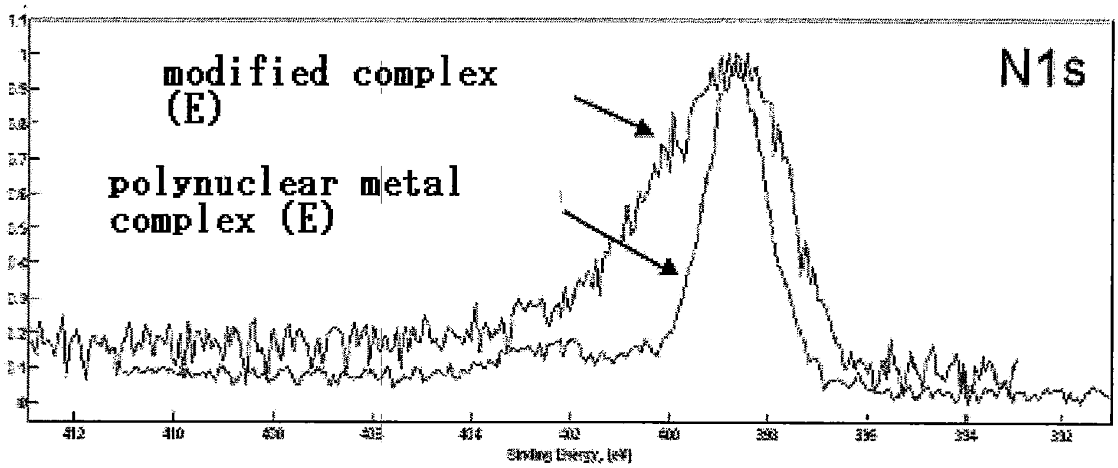
N1s
modified complex
(E)
polynuclear metal
complex (E)

Fig. 1 2

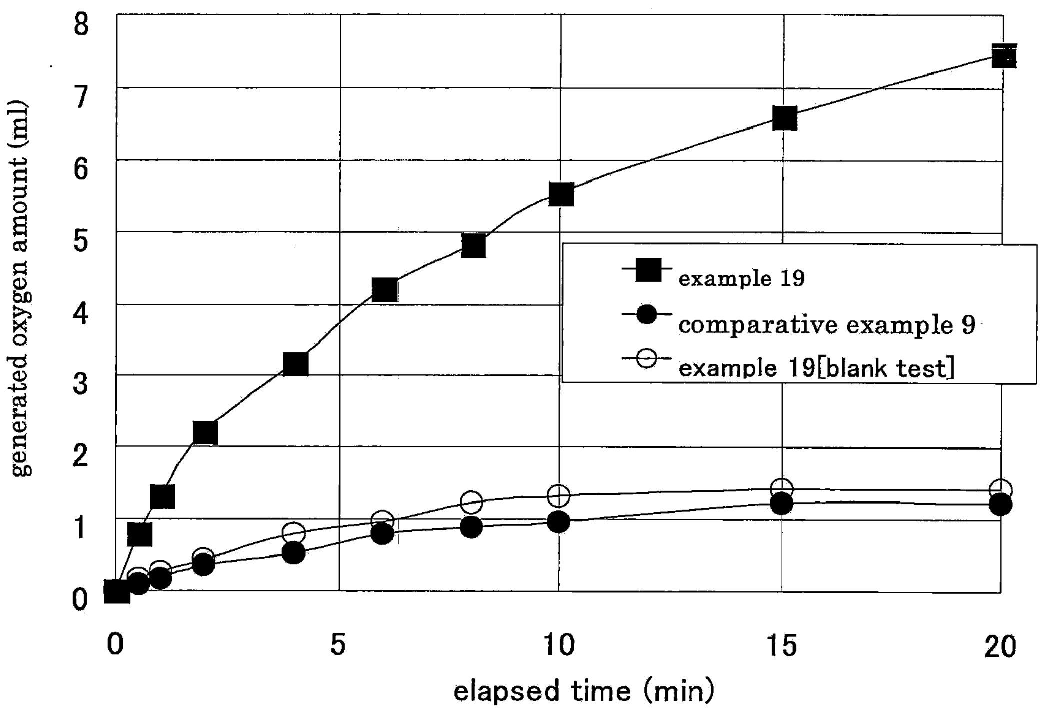
generated oxygen amount (ml)
0
1
2
3
4
5
6
7
8
0
5
10
15
20
elapsed time (min)
example 19
comparative example 9
example 19[blank test]

Fig. 1 3

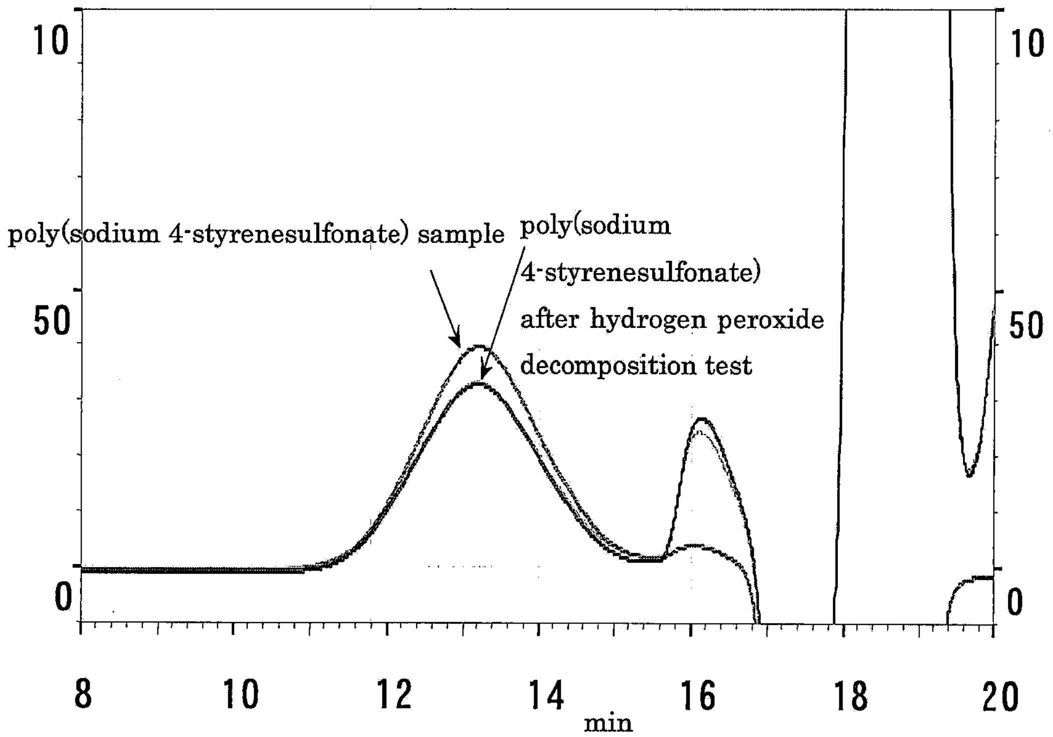
10
50
0
10
50
0
poly(sodium 4-styrenesulfonate) sample
poly(sodium
4-styrenesulfonate)
after hydrogen peroxide
decomposition test
8
10
12
14
16
18
20
min

## INTERNATIONAL SEARCH REPORT

International application No.
PCT/JP2007/052157

A. CLASSIFICATION OF SUBJECT MATTER
*C07D257/10*(2006.01)i, *B01J31/22*(2006.01)i, *B01J37/08*(2006.01)i, *B01J37/34*(2006.01)i, *C07F1/08*(2006.01)n, *C07F9/00*(2006.01)n, *C07F13/00*(2006.01)n, *C07F15/02*(2006.01)n, *C07F15/04*(2006.01)n, *C07F15/06*(2006.01)n

According to International Patent Classification (IPC) or to both national classification and IPC

B. FIELDS SEARCHED

Minimum documentation searched (classification system followed by classification symbols)
C07D257/10, B01J31/22, B01J37/08, B01J37/34, C07F1/08, C07F9/00, C07F13/00, C07F15/02, C07F15/04, C07F15/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| A | Shigeo KIDA et al., "Fukukaku Kinzoku Sakutai no Kozo to Shokubai Sayo", Shokubai, 1990, Vol.32, No.1, p.14-24 | 1-14 |
| A | Minori UEHARA et al., High Activities of Hydrogen Peroxide Decomposition Catalyzed by Dinuclear Schiff Base Mn(III)-Cu(II) Complexes Accompanying Metal Substitution in N,N-Dimethylformamide and the Crystal Structure of a Substituted Schiff Base Cu(II) Complex, Bulletin of the Chemical Society of Japan, 1998, Vol.71, No.5, p.1081-1088 | 1-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 February, 2007 (26.02.07) | 06 March, 2007 (06.03.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2007/052157

C (Continuation). DOCUMENTS CONSIDERED TO BE RELEVANT

| Category* | Citation of document, with indication, where appropriate, of the relevant passages | Relevant to claim No. |
|---|---|---|
| A | Erlund J. LARSON et al., Catalytic Disproportionation of Hydrogen Peroxide by [MnIV(μ2-O)(SALPN)]2, Journal of the American Chemical Society, 1991, Vol.113, No.20, p.7809-7810 | 1-14 |
| A | WO 2005-095408 A1 (Nagoya Institute of Technology), 13 October, 2005 (13.10.05), Full text (Family: none) | 1-14 |
| A | JP 09-324043 A (Sumitomo Chemical Co., Ltd.), 16 December, 1997 (16.12.97), Full text (Family: none) | 1-14 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7068171 A
- JP 2002104811 A


### Non-patent literature cited in the description


- **KENICHI OYAIZU ; MAKOTO YUASA.** *Surface,* 2003, vol. 41 (3), 22
- **MICHINORI OKI et al.** ENCYCLOPAEDIA CHIMICA. 01 July 2005
- **A. E. BOELRIJK ; G. C. DISMUKES.** *Inorg. Chem.,* 2000, vol. 39, 3020
- **RYOGO KUBO et al.** Encyclopedia of Physics and Chemistry. Iwanami Shoten, 10 January 1991, 966
- **MICHINORI OKI et al.** ENCYCLOPAEDIA CHIMICA. Tokyo Kagaku Dojin, 01 July 2005, 1323-1324
- Base of Carbonization Engineering. Ohmsha, Ltd, 1982
- *Chemical Communications,* 2005, 3385
- *Bulletin of Chemical Society of Japan,* 1995, vol. 68, 1105
- *Australian Journal of Chemistry,* 1970, vol. 23, 2225
- *Chemische Berichte,* 1994, vol. 127, 465
- *Journal of Chemical Society, Dalton Transactions,* 1996, 1223